(19) Europäisches Patentamt
European Patent Office
Office européen des brevets

(11) Veröffentlichungsnummer: **0 278 911 B1**

(12) **EUROPÄISCHE PATENTSCHRIFT**

(45) Veröffentlichungstag der Patentschrift: **04.08.93**

(51) Int. Cl.$^5$: **C07D 499/00**, A61K 31/43, //C07F9/568

(21) Anmeldenummer: **88810069.0**

(22) Anmeldetag: **05.02.88**

(54) **Bicyclische beta-Lactam-carbonsäuren.**

(30) Priorität: **11.02.87 CH 498/87**
**03.09.87 CH 3373/87**

(43) Veröffentlichungstag der Anmeldung:
**17.08.88 Patentblatt 88/33**

(45) Bekanntmachung des Hinweises auf die Patenterteilung:
**04.08.93 Patentblatt 93/31**

(84) Benannte Vertragsstaaten:
**AT BE CH DE ES FR GB GR IT LI LU NL SE**

(56) Entgegenhaltungen:
**EP-A- 0 070 204**
**EP-A- 0 170 028**
**EP-A- 0 236 880**
**DE-A- 2 950 898**

(73) Patentinhaber: **CIBA-GEIGY AG**
**Klybeckstrasse 141**
**CH-4002 Basel(CH)**

(72) Erfinder: **Lang, Marc, Dr.**
**Rue des Ormes 6**
**F-68170 Rixheim(FR)**

EP 0 278 911 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue bicyclische beta-Lactam-carbonsäuren der Formel

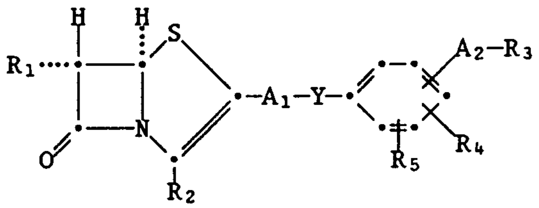

(I),

worin $R_1$ Hydroxymethyl oder 1-Hydroxyäthyl ist, $R_2$ Carboxy oder unter physiologischen Bedingungen spaltbares verestertes Carboxy bedeutet, $R_3$ Amino, Methylamino, Dimethylamino, (2-Hydroxyäthyl)-amino, Bis-(2-hydroxyäthyl)-amino, (4-Pyridyl)-amino, (3-Pyridyl)-amino, Morpholin-4-yl, Piperazin-1-yl, 4-Carbamoyl-piperazin-1-yl, 3-Hydroxy-4-oxo-1,4-dihydro-pyridin-1-yl, 1H-Tetrazol-1-yl, Pyridin-4-ylthio oder Carbamoyl ist, $R_4$ und $R_5$ Wasserstoff bedeuten, Y für eine Gruppe -O- steht, $A_1$ $C_1$-$C_2$-Alkylen ist und $A_2$ für eine direkte Bindung steht oder $C_1$-$C_2$-Alkylen bedeutet, die reinen optischen Isomere von Verbindungen der Formel I und Mischungen dieser optischen Isomere, Salze von Verbindungen der Formel I, die eine salzbildende Gruppe aufweisen, mit Ausnahme von quaternären Ammoniumsalzen, Verfahren zur Herstellung von Verbindungen der Formel I, pharmazeutische Präparate, die solche Verbindungen enthalten, und ihre Verwendung zur Herstellung von pharmazeutischen Präparaten oder als Arzneimittelwirkstoffe.

Die EP-A-236 880 stellt im Umfang des darin beschriebenen Gegenstands, welcher bereits in der entsprechenden Prioritätsanmeldung offenbart ist, ein älteres Recht im Sinne von Art. 54 (3) EPUe in allen in der vorliegenden Anmeldung benannten Vertragsstaaten mit Ausnahme von Luxemburg dar. In diesem älteren Recht sind Penemderivate der obenganannten Formel I beschrieben, die sich von den Verbindungen der vorliegenden Anmeldung insbesondere hinsichtlich der Definition des Restes $R_3$ unterscheiden, welcher die Position des im älteren Recht angeführten Restes Q einnimmt.

Die vor- und nachstehend verwendeten Definitionen haben im Rahmen der vorliegenden Beschreibung vorzugsweise die folgenden Bedeutungen:
Eine unter physiologischen Bedingungen spaltbare (d.h. metabolisierbare) veresterte Carboxylgruppe $R_2$ ist in erster Linie eine Acyloxymethoxycarbonylgruppe oder auch Acylaminomethoxycarbonylgruppe, worin Acyl z.B. der Rest einer organischen Carbonsäure, in erster Linie einer gegebenenfalls, z.B. durch Amino, substituierten Niederalkancarbonsäure oder Arencarbonsäure, z.B. Benzoesäure, ist oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere $\alpha$-Aminoniederalkanoyloxymethoxycarbonyl, und Niederalkanoylaminomethoxycarbonyl. Weitere unter physiologischen Bedingungen spaltbare veresterte Carboxylgruppen $R_2$ sind z.B. 3-Phthalidyloxycarbonyl, 1-Niederalkoxycarbonyloxyniederalkoxycarbonyl, 1-Niederalkoxyniederalkoxycarbonyl oder 2-Oxo-1,3-dioxolen-4-ylmethoxycarbonyl, welches in 5-Stellung des Dioxolenringes gegebenenfalls durch Niederalkyl oder Phenyl substituiert ist.

In der vorliegenden Beschreibung bedeutet der im Zusammenhang mit Definitionen von Gruppen bzw. Verbindungen verwendete Ausdruck "Nieder", das die entsprechenden Gruppen bzw. Verbindungen, sofern nicht ausdrücklich anders definiert, 1 bis 7, bevorzugt 1 bis 4 Kohlenstoffatome enthalten.

Niederalkanoyloxymethoxycarbonyl ist z.B. Acetoxymethoxycarbonyl oder Pivaloyloxymethoxycarbonyl. $\alpha$-Aminoniederalkanoyloxymethoxycarbonyl ist z.B. Glycyloxymethoxycarbonyl, L-Valyloxymethoxycarbonyl oder L-Leucyloxymethoxycarbonyl. Niederalkanoylaminomethoxycarbonyl ist z.B. Acetaminomethoxycarbonyl. 1-Niederalkoxycarbonyloxyniederalkoxycarbonyl ist z.B. Aethoxycarbonyloxymethoxycarbonyl oder 1-Aethoxycarbonyloxyäthoxycarbonyl. 1-Niederalkoxyniederalkoxycarbonyl ist z.B. Methoxymethoxycarbonyl oder 1-Methoxyäthoxycarbonyl. Bei einer 2-Oxo-1,3-dioxolen-4-ylmethoxy-Gruppe, welche in 5-Stellung des Dioxolen-Ringes gegebenenfalls durch Niederalkyl oder Phenyl substituiert ist, handelt es sich vor allem um eine 5-Phenyl- und in erster Linie um eine 5-Methyl-2-oxo-1,3-dioxolen-4-ylmethoxy-Gruppe.

$C_2$-Alkylen $A_1$ und $A_2$ ist insbesondere geradkettig, d.h. Aethylen, kann aber auch verzweigtkettig sein, d.h. Aethyliden.

Bevorzugt als Rest $R_1$ ist 1-Hydroxyäthyl. Bevorzugte unter physiologischen Bedingungen spaltbare veresterte Carboxylgruppen $R_2$ sind z.B. Niederalkanoyloxymethoxycarbonyl, z.B. Acetoxymethoxycarbonyl oder Pivaloyloxymethoxycarbonyl, und 1-Niederalkoxycarbonyloxyniederalkoxycarbonyl,z.B. 1-Aethoxycarbonyloxy-äthoxycarbonyl. Die Gruppierung $-A_2-R_3$ ist in o-, m- oder insbesondere p-Stellung

zum Rest Y angeordnet ist.

In den Ausgangsstoffen zur Herstellung der Verbindungen der Formel I sind die darin enthaltenen funktionellen Gruppen, wie Hydroxy-, Carboxy- oder Aminogruppen, gegebenenfalls durch konventionelle Schutzgruppen geschützt, die in der Penem-, Penicillin-, Cephalosporin- und Peptidchemie verwendet werden. Solche Schutzgruppen schützen die betreffenden funktionellen Gruppen vor unerwünschten Kondensationsreaktionen, Substitutionsreaktionen und dergleichen wahrend der Synthese der Verbindung der Formel I aus ihren Vorstufen. Solche Schutzgruppen sind leicht, d.h. ohne dass unerwünschte Nebenreaktionen stattfinden, beispielsweise solvolytisch oder reduktiv abspaltbar.

Schutzgruppen dieser Art sowie ihre Einführung und Abspaltung sind beispielsweise beschrieben in
J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In den Ausgangsstoffen zur Herstellung von Verbindungen der Formel (I) kann eine Hydroxygruppe beispielsweise durch einen Acylrest geschützt sein. Geeignete Acylreste sind z.B. gegebenenfalls durch Halogen substituiertes Niederalkoxycarbonyl, z.B. 2-Bromäthoxycarbonyl oder 2,2,2-Trichloräthoxycarbonyl, Niederalkenyloxycarbonyl, z.B. Allyloxycarbonyl, Niederalkenyloxyoxalyl, z.B. Allyloxyoxalyl, oder gegebenenfalls durch Nitro substituiertes Phenylniederalkoxycarbonyl, z.B. Benzyloxycarbonyl oder 4-Nitrobenzyloxycarbonyl. Weitere geeignete Hydroxyschutzgruppen sind z.B. trisubstituiertes Silyl, wie Triniederalkylsilyl, z.B. Trimethylsilyl, Dimethyl-(2,3-dimethylbut-2-yl)-silyl oder tert.-Butyldimethylsilyl. Bevorzugt als Hydroxyschutzgruppe sind Triniederalkylsilyl, Niederalkenyloxyoxalyl und Niederalkenyloxycarbonyl.

Eine Carboxylgruppe ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist.

Solche veresterten Carboxylgruppen enthalten als veresternde Gruppen in erster Linie in 1-Stellung verzweigte oder in 1- oder 2-Stellung geeignet substituierte Niederalkylgruppen. Geeignete in veresterter Form vorliegende Carboxylgruppen sind unter anderen gegebenenfalls durch Nitro oder Niederalkoxy, wie Methoxy, substituiertes Benzyloxycarbonyl, z.B. 4-Nitrobenzyloxycarbonyl oder 4-Methoxybenzyloxycarbonyl, Niederalkanoylmethoxycarbonyl, wie Acetonyloxycarbonyl, Halogenniederalkoxycarbonyl, wie 2-Halogenniederalkoxycarbonyl, z.B. 2,2,2-Trichloräthoxycarbonyl oder 2-Bromäthoxycarbonyl, Niederalkenyloxycarbonyl, z.B. Allyloxycarbonyl, oder in 2-Stellung durch Triniederalkylsilyl substituiertes Aethoxycarbonyl, z.B. 2-Trimethylsilyläthoxycarbonyl oder 2-(Di-n-butyl-methyl-silyl)-äthoxycarbonyl. Bevorzugte geschützte Carboxylgruppen sind die 4-Nitrobenzyloxycarbonyl-, Niederalkenyloxycarbonyl-, insbesondere Allyloxycarbonyl- und die in 2-Stellung durch Triniederalkylsilyl, z.B. Trimethylsilyl oder Di-n-butyl-methylsilyl, substituierte Aethoxycarbonyl-Gruppe.

Eine geschützte Amino-Gruppe kann beispielsweise in Form einer leicht spaltbaren Acylaminogruppe, Nitrogruppe oder auch als Azidogruppe vorliegen. In einer entsprechenden Acylaminogruppe ist Acyl beispielsweise der Acylrest einer gegebenenfalls z.B. durch Halogen oder Phenyl, substituierten Niederalkancarbonsäure oder insbesondere eines Kohlensäurehalbesters. Solche Acylgruppen sind beispielsweise Niederalkenyloxycarbonyl, z.B. Allyloxycarbonyl, gegebenenfalls substituiertes Benzyloxycarbonyl, z.B. 4-Nitrobenzyloxycarbonyl, oder 2-Halogenniederalkoxycarbonyl, z.B. 2,2,2-Trichloräthoxycarbonyl oder 2-Bromäthoxycarbonyl, oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl. Bevorzugte geschützte Aminogruppen sind z.B. Azido, Nitro, Niederalkenyloxycarbonylamino, z.B. Allyloxycarbonylamino, und gegebenenfalls durch Nitro substituiertes Benzyloxycarbonylamino.

Salze von erfindungsgemässen Verbindungen sind in erster Linie pharmazeutisch annehmbare, nichttoxische Salze von Verbindungen der Formel I. Solche Salze werden von der in den Verbindungen der Formel I vorhandenen sauren Carboxylgruppe $R_2$ gebildet und sind in erster Linie Metall- oder Ammoniumsalze, wie Alkalimetall- und Erdalkalimetall-, z.B. Natrium-, Kalium-, Magnesium - oder Calciumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethyl-piperidin, Cycloalkylaminen, z.B. Dicyclohexylamin, oder Benzylaminen, z.B. N,N′-Dibenzyläthylendiamin, Dibenzylamin oder N-Benzyl-$\beta$-phenäthylamin. Verbindungen der Formel I mit einer basischen Gruppe, z.B. mit einer Aminogruppe $R_3$, können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure, bilden. Verbindungen der Formel I mit einer sauren und mit einer basischen Gruppe können auch in Form von inneren Salze, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Penem-Verbindungen der Formel I können im Rest $R_1$ ein zusätzliches Chiralitätszentrum besitzen. 1-Hydroxyäthyl als Rest $R_1$ kann in der R-, in der S- oder in der racemischen R,S-Konfiguration vorliegen. In bevorzugten Penem-Verbindungen der Formel I weist 1-Hydroxyäthyl $R_1$ die R-Konfiguration auf. Die Erfindung betrifft demgemäss die reinen Diastereomeren und Mischungen der Diastereomeren von Verbindungen der Formel I, welche im Rest $R_1$ zusätzliche Chiralitätszentren aufweisen.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin funktionelle Gruppen in ungeschützter Form vorliegen, und pharmazeutisch annehmbare Salze von solchen Verbindungen mit salzbildenden Gruppen haben antibakterielle Wirkungen. Beispielsweise sind sie in vitro gegen grampositive und gramnegative Kokken, inklusive Methicillinresistente Staphylokokken, z.B. Staphylococcus aureus, Staphylococcus epidermidis, Streptococcus pyogenes, Streptococcus pneumoniae und Streptococcus faecalis, und Haemophilus influenzae in minimalen Konzentrationen von <0,01 bis ca. 8 µg/ml und gegen Enterobacteriaceae, z.B. Escherichia coli und Klebsiella pneumoniae, Pseudomonas aeruginosa und Anaerobier, z.B. Bacteroides sp., in minimalen Konzentrationen von 0,1 bis ca. 64 µg/ml wirksam. In vivo, bei der systemischen Infektion der Maus, z.B. durch Staphylococcus aureus und Escherichia coli ergeben sich bei subkutaner oder oraler Applikation erfindungsgemässer Verbindungen $ED_{50}$-Werte von ca. 0,2 bis ca. 30 mg/kg.

Die neuen Verbindungen können als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, können als Zwischenprodukte zur Herstellung der oben genannten pharmakologisch wirksamen Verbindungen der Formel I verwendet werden.

Die Erfindung betrifft hauptsächlich Verbindungen der Formel I, worin $R_1$ Hydroxymethyl oder 1-Hydroxyäthyl ist, $R_2$ Carboxy oder unter physiologischen Bedingungen spaltbares verestertes Carboxy bedeutet, $R_3$ Amino oder Carbamoyl ist, $R_4$ und $R_5$ Wasserstoff bedeuten, Y für eine Gruppe -O- steht, $A_1$ Alkylen mit 1 oder 2 Kohlenstoffatomen ist und $A_2$ für eine direkte Bindung steht oder Alkylen mit 1 oder 2 Kohlenstoffatomen bedeutet, die reinen optischen Isomere von Verbindungen der Formel I und Mischungen dieser optischen Isomere, und Salze von Verbindungen der Formel I, die eine salzbildende Gruppe aufweisen.

Die Erfindung betrifft vorzugsweise Verbindungen der Formel I, worin $R_1$ 1-Hydroxyäthyl ist, $R_2$ Carboxy ist, $R_3$ Amino, Methylamino oder Dimethylamino bedeutet, $R_4$ und $R_5$ jeweils Wasserstoff bedeuten, Y für eine Gruppe -O- steht, $A_1$ Methylen ist und $A_2$ Methylen oder Aethylen ist, die reinen optischen Isomere von Verbindungen der Formel I und Mischungen dieser optischen Isomere, und Salze davon.

Die Erfindung betrifft in erster Linie die in den Beispielen genannten Verbindungen der Formel I und ihre pharmazeutisch annehmbaren Salze.

Die Verbindungen der vorliegenden Erfindung können nach an sich bekannten Verfahren hergestellt werden.

Die neuen Verbindungen werden z.B. hergestellt, indem man

a) in einer Verbindung der Formel

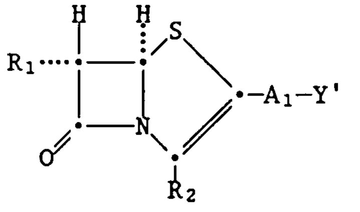

(II),

worin $R_1$, $R_2$ und $A_1$ die unter Formel (I) angegebenen Bedeutungen haben, und Y' eine in den Rest

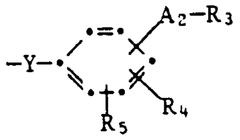

(IIa),

worin Y, $A_2$, $R_3$, $R_4$ und $R_5$ die unter Formel (I) angegebenen Bedeutungen haben, überführbare Gruppe ist, die Gruppe Y′ in den Rest

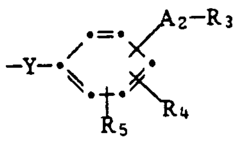

(IIa)

überführt, oder

b) eine Ylid-Verbindung der Formel

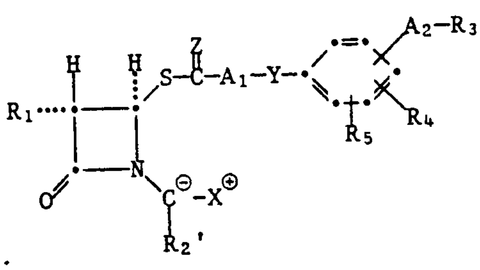

(III),

worin $R_1$, $R_3$, $R_4$, $R_5$, $A_1$, $A_2$ und Y die unter Formel I angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

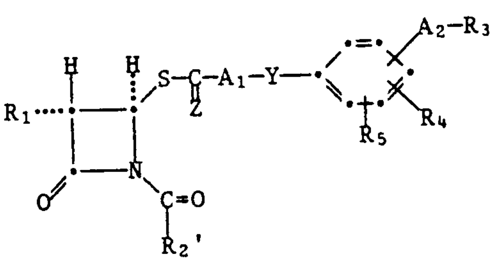

(IV),

worin $R_1$, $R_3$, $R_4$, $R_5$, $A_1$, $A_2$ und Y die unter Formel I angegebenen Bedeutungen haben, Z Sauerstoff oder Schwefel ist und $R_2'$ eine geschützte Carboxylgruppe ist, mit einer organischen Verbindung des dreiwertigen Phosphors behandelt, und wenn erwünscht oder notwendig, in einer erhältlichen Verbindung der Formel I eine geschützte funktionelle Gruppen in die freie funktionelle Gruppe überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

In den Ausgangsverbindungen der Formeln II-IV sind funktionelle Gruppen, wie freie Hydroxygruppen, z.B. im Rest $R_1$, und freie Aminogruppen vorzugsweise durch konventionelle Schutzgruppen, z.B. durch eine der oben genannten, geschützt.

a) Umsetzung der Verbindung der Formel II

Eine in den Rest

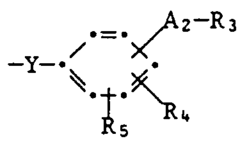

(IIa)

überführbare Gruppe Y′ ist beispielsweise Hydroxy oder eine durch nucleophile Reaktion austauschbare Gruppe.

So können Verbindungen der Formel I, worin Y eine Gruppe -O- ist, hergestellt werden, indem man eine Verbindung der Formel II, worin Y′ für Hydroxy steht, mit einem Phenol der Formel

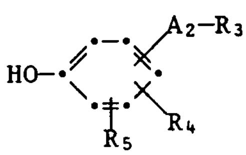

(Va)

in Gegenwart einer organischen Verbindung des dreiwertigen Phosphors, wie eines Triniederalkylphosphins, z.B. Tri-n-butyl-phosphin, eines Triniederalkylphosphits, z.B. Triäthylphosphit, oder Triphenylphosphin, und eines Azodicarbonsäurediesters, wie eines entsprechenden Triniederalkylesters, z.B. Azodicarbonsäurediäthylester, in einem inerten aprotischen Lösungsmittel, wie in einem cyclischen Aether, z.B. Tetrahydrofuran oder Dioxan, bei Raumtemperatur oder erniedrigter oder etwas erhöhter Temperatur, z.B. in einem Temperaturbereich von ca. -70° bis ca. 40°C, bevorzugt bei etwa 0° bis etwa 20°C, umsetzt.

b) Cyclisierung der Verbindung der Formel III

Die Gruppe $X^{\oplus}$ im Ausgangsmaterial der Formel III ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetallion, z.B. das Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. dem Natriumion.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 80°C bis etwa 130°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, und, falls notwendig, in einer Inertgas-, z.B. Stickstoffatmosphäre, durchgeführt.

c. Cyclisierung der Verbindung der Formel IV

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)_2N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten

Kohlenwasserstoff, z.B. Methylenchlorid oder Chloroform, bei einer Temperatur von etwa 20° bis 140°C, bevorzugt bei etwa 40° bis etwa 110°C, durchgeführt, wobei man 1 Moläquivalent einer Verbindung der Formel IV mit 2 Moläquivalenten der Phosphorverbindung umsetzt. Vorzugsweise legt man die Verbindung der Formel IV in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

In einer bevorzugten Ausführungsform des Verfahrens stellt man das Ausgangsmaterial der Formel IV wie weiter unten angegeben her und setzt es ohne Isolierung aus dem Reaktionsgemisch mit der organischen Verbindung des dreiwertigen Phosphors um, wobei die Endprodukte der Formel I entstehen.

Bevorzugt werden solche Ausgangsmaterialien der Formeln II-V verwendet, die zu den eingangs als besonders bevorzugt genannten Verbindungen der Formel I führen.

In einer erhältlichen Verbindung der Formel I, worin eine oder mehrere funktionelle Gruppen geschützt sind, können diese, z.B. geschützte Carboxyl-, Hydroxy- und/oder Aminogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer verfahrensgemäss erhältlichen Verbindung der Formel I, worin $R_2$ eine geschützte Carboxylgruppe bedeutet, kann die geschützte Carboxylgruppe in an sich bekannter Weise freigesetzt werden. So kann man in 2-Stellung durch eine trisubstituierte Silylgruppe substituiertes Aethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)-palladium, gegebenenfalls in Gegenwart von Triphenylphosphin und unter Zusatz eines Allylgruppenakzeptors, wie einer Carbonsäure, z.B. 2-Aethylhexansäure, oder eines Salzes davon oder Dimedon oder Tributylzinnhydrid, erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) in freies Carboxyl umwandeln. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl übergeführt werden.

Andererseits können auch Verbindungen der Formel I, worin $R_2$ Carboxyl, bedeutet, in Verbindungen der Formel I überführt werden, worin $R_2$ eine unter physiologischen Bedingungen spaltbare veresterte Carboxylgruppe darstellt. So kann man die freie Carboxylgruppe z.B. durch Umsetzen mit einem entsprechenden Alkohol in Gegenwart eines Veresterungsmittels, wie eines Carbodiimids, z.B. Dicyclohexylcarbodiimid, oder Carbonyldiimidazol, verestern. Solche Ester können auch durch Umsetzung eines gegebenenfalls in situ hergestellten Salzes der Säure mit dem reaktionsfähigen Ester eines entsprechenden Alkohols und einer starken anorganischen Säure, wie Schwefelsäure, oder einer starken organischen Sulfonsäure, wie 4-Toluolsulfonsäure, hergestellt werden.

In verfahrensgemäss erhältlichen Verbindungen der Formel I, worin der Rest $R_1$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silylgruppe geschützte Hydroxygruppe wie eine entsprechend geschützte Aminogruppe freigesetzt (s.u.); eine Triniederalkylsilylgruppe kann z.B. auch mit Tetrabutylammoniumfluorid und Essigsäure abgespalten werden (unter diesen Bedingungen werden durch trisubstituiertes Silyläthoxy geschützte Carboxygruppen nicht gespalten).

In einer erfindungsgemäss erhältlichen Verbindung der Formel I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe) oder 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators gespalten werden. 4-Nitrobenzyloxycarbonylamino kann auch durch Behandeln mit einem Alkalimetall-, z.B. Natriumdithionit gespalten werden. Allyloxycarbonylamino kann durch Umsetzen mit einer Verbindung des Palladiums, z.B. Tetrakis-(triphenylphosphin)-palladium, gegebenenfalls in Gegenwart von Triphenylphosphin und in Gegenwart eines Allylgruppenakzeptors, wie einer Carbonsäure, z.B. 2-Aethylhexansäure, oder eines Salzes davon oder Dimedon oder Tributylzinnhydrid, gespalten werden. Eine durch 2-substituiertes Silyläthoxycarbonyl geschützte Aminogruppe kann durch Behandeln mit einem Fluoridanion liefernden Salz der Fluorwasserstoffsäure, wie einem Alkalimetallfluorid,
z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Nitro- oder Azidogruppe geschützte Aminogruppe wird z.B. durch Reduktion in freies Amino überführt, beispielsweise durch katalytische Hydrierung mit Wasserstoff in Gegenwart eines Hydrierkatalysators, wie Platinoxid oder Palladium, oder durch Behandeln mit Zink in Gegenwart einer Säure, wie Essigsäure.

Weiterhin kann man in verfahrensgemäss erhältlichen Verbindungen der Formel I einen Rest $R_3$ in einen anderen Rest $R_3$ umwandeln.

Beispielsweise kann in einer Verbindung der Formel I, worin $R_3$ Amino ist, die Aminogruppe auch in eine Dimethylaminogruppe überführt werden, beispielsweise durch Umsetzung mit Formalin und einem Lithium- oder Bor-Hydrid, wie Natriumcyanoborhydrid.

Salze von Verbindungen der Formel I mit salzbildenden Gruppen können in an sich bekannter Weise hergestellt werden. So kann man Salze von Verbindungen der Formel I mit einer freien Carboxylgruppe z.B. durch Behandeln mit Metallverbindungen, wie anorganischen Alkali- oder Erdalkalimetallsalzen, z.B. Natriumhydrogencarbonat, oder mit Ammoniak oder mit einem geeigneten organischen Amin herstellen, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von isomeren Verbindungen können nach an sich bekannten Methoden in die einzelnen Isomere aufgetrennt werden. Beispielsweise kann man ein erhaltenes Racemat mit einem optisch aktiven Hilfsstoff reagieren lassen, das dabei entstandene Gemisch zweier diastereomerer Verbindungen mit Hilfe von geeigneten physikalisch-chemischenMethoden (z.B. fraktioniertes Kristallisieren, Adsorptionschromatographie) trennen und die einzelnen diastereomeren Verbindungen dann in die optisch aktiven Verbindungen spalten.

Bei allen nachträglichen Umwandlungen erhaltener Verbindungen der Formel I werden solche Reaktionen bevorzugt, die unter gemässigt alkalischen oder insbesondere neutralen Bedingungen erfolgen.

Das Verfahren umfasst auch diejenigen Ausführungsformen, wonach Ausgangstoffe in Form von Derivaten verwendet oder in situ, gegebenenfalls unter den Reaktionsbedingungen, hergestellt werden.

Die Ausgangsverbindungen der Formeln III und IV können, wie in dem folgenden Reaktionsschema I angegeben, hergestellt werden:

Reaktionsschema I

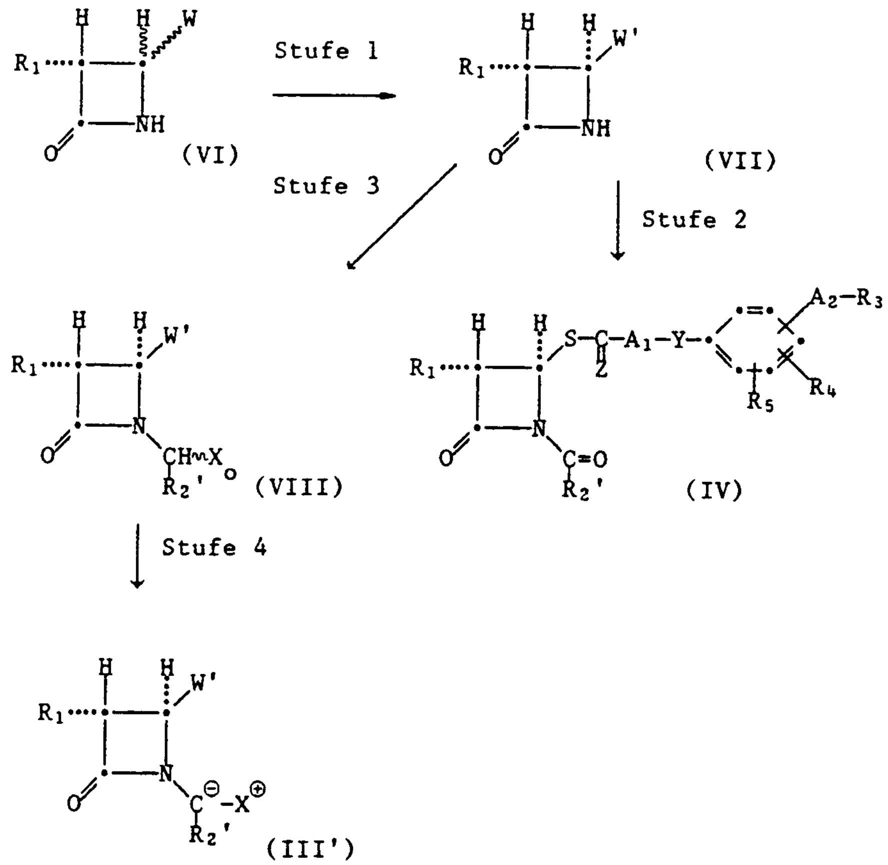


In den Verbindungen der Formeln III′, VII und VIII steht W′ für den Rest

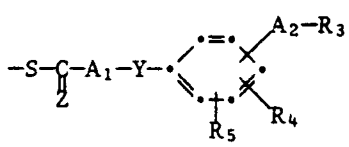

oder für Triphenylmethylthio oder Niederalkanoylthio.

Stufe 1

Geeignete Ausgangsverbindungen der Formel VI, worin W ein durch nucleophile Reaktion leicht austauschbarer Rest, z.B. Niederalkanoyloxy, wie Acetyloxy, oder Sulfonyloxy $R_o$-$SO_2$-, worin $R_o$ z.B. gegebenenfalls durch Hydroxy substituierten Niederalkyl, wie Methyl, tert.Butyl oder 2-Hydroxyäthyl, ist, sind beispielsweise aus der veröffentlichten Europäischen Patentanmeldung Nr. 82113, der Deutschen Offenlegungsschrift Nr. 3 224 055 und der Deutschen Offenlegungsschrift Nr. 3 013 997 bekannt oder können in dazu analoger Weise hergestellt werden.

Eine den Rest

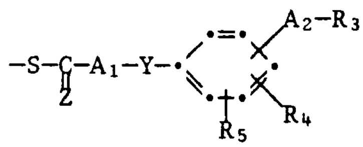

einführende Verbindung ist beispielsweise eine Säure der Formel

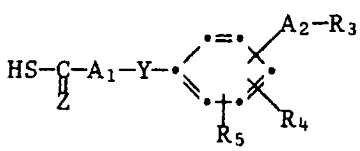

(IXa)

oder insbesondere ein Salz, z.B. ein Alkalimetallsalz, wie das Natrium- oder Kaliumsalz, davon. Die Substitution kann in einem organischen Lösungsmittel, wie in einem Niederalkanol, einem Niederalkancarbonsäureamid, einem cyclischen Aether oder in einem ähnlichen inerten Lösungsmittel bei Raumtemperatur oder bei etwas erhöhter oder erniedrigter Temperatur, z.B. bei etwa 0° bis etwa 40°C, durchgeführt werden. Die Einführung eines Triphenylmethylthio- oder Niederalkanoylthio-Restes $W'$ erfolgt in analoger Weise durch Umsetzen mit einem Alkalimetallsalz, z.B. dem Natriumsalz, einer Niederalkanthiocarbonsäure, z.B. Thioessigsäure, oder des Triphenylmethylmercaptans.

Stufe 2

Eine Ausgangsverbindung der Formel (IV) wird erhalten, indem man ein Azetidinon der Formel (VII), in der $W'$ für den Rest

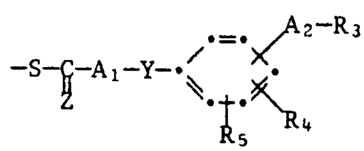

steht, mit einer Säure der Formel $R_2'$-COOH oder insbesondere einem reaktionsfähigen Derivat, wie einem Ester oder Säurehalogenid, z.B. dem Säurechlorid, davon bei einer Temperatur von -50° bis 80°C, bevorzugt bei -20° bis 0°C, in einem inerten Lösungsmittel, wie einem der bei der Umsetzung von Verbindungen der Formel (IV) zu Verbindungen der Formel I genannten, behandelt. Bei Verwendung eines Säurehalogenids arbeitet man vorzugsweise in Gegenwart eines säurebindenden Mittels wie eines tertiären aliphatischen Amins, eines aromatischen Amins oder insbesondere eines Alkalimetall- oder Erdalkalimetallcarbonats oder -hydrogencarbonats.

Verbindungen der Formel (VII), worin $W'$ für Triphenylmethylthio oder Niederalkanoylthio steht, können in die Ausgangsverbindungen der Formel VII, worin $W'$ für den Rest

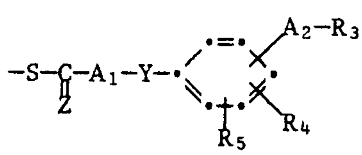

steht, überführt werden, indem man sie in Gegenwart einer Base, z.B. Pyridin oder Tri-n-butylamin, in einem geeigneten Lösungsmittel, z.B. Diäthyläther oder Methanol, mit einem Salz der Formel MA, worin M für ein Uebergangsmetallkation, insbesondere für das Silberkation, steht, und A ein gebräuchliches Anion

darstellt, welches die Löslichkeit des Salzes MA in dem gewählten Lösungsmittel begünstigt, z.B. das Nitrat-, Acetat- oder Fluorid-Anion, umsetzt, und das entstandene Salz der Formel

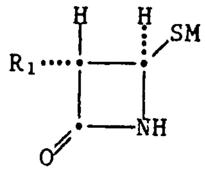

(VII'),

mit einem den Rest

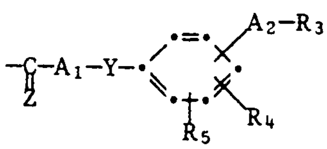

einführenden Acylierungsmittel, z.B. mit der Säure

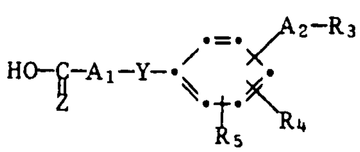

(IXb)

oder einem reaktionsfähigen funktionellen Derivat, wie einem Säurehalogenid, z.B. dem Chlorid oder Bromid, Azid oder Anhydrid davon, behandelt. Die Acylierung erfolgt, wenn ein reaktionsfähiges funktionelles Derivat der Säure der Formel (IXb), z.B. das Säurechlorid, eingesetzt wird, in einem inerten Lösungsmittel, wie einem chlorierten Kohlenwasserstoff, oder einem Aether, bei Raumtemperatur oder unter Erhitzen oder Kühlen, z.B. in einem Temperaturbereich von ca. -50° bis ca. +60°C, insbesondere bei ca. -30° bis ca. +20°C.

Stufe 3

Verbindungen der Formel VIII, worin $X_o$ für eine reaktionsfähige veresterte Hydroxygruppe, insbesondere für Halogen, z.B. Chlor oder Brom, steht, werden hergestellt, indem man eine Verbindung der Formel VII mit einer Glyoxylsäure-Verbindung der Formel $R_2'$-CHO oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel VIII, worin $X_o$ für Hydroxy steht, die Hydroxygruppe in eine reaktionsfähige veresterte Hydroxygruppe umwandelt.

Die Verbindungen der Formel VIII werden üblicherweise als Gemisch der beiden Isomere [bezüglich der Gruppierung -CH($R'_2$)～$X_o$] erhalten.

Die Anlagerung der Glyoxylsäureester-Verbindung an das Stickstoffatom des Lactamrings in der Verbindung der Formel VII findet bei Raumtemperatur oder, falls notwendig, unter Erwärmen, statt. Bei Verwendung des Hydrats der Glyoxylsäure-Verbindung wird Wasser gebildet, das, wenn notwendig, durch Destillation, z.B. azeotrop, oder durch Verwendung eines geeigneten Dehydratationsmittels enfernt wird. Vorzugsweise arbeitet man in Gegenwart eines geeigneten inerten Lösungsmittels oder Lösungsmittelgemisches.

Die Ueberführung einer Hydroxygruppe $X_o$ in eine reaktionsfähige veresterte Hydroxygruppe $X_o$ in einer Verbindung der Formel VIII wird durch Behandeln mit einem geeigneten Veresterungsmittel durchgeführt, z.B. mit einem Thionylhalogenid, z.B. -chlorid, vorzugsweise in Gegenwart eines basischen, in erster Linie organischen basischen Mittels, wie eines aliphatischen tertiären Amins, oder einer heterocyclischen Base vom Pyridintyp. Vorzugsweise arbeitet man in Gegenwart eines geeigneten Lösungsmittels, z.B. Dioxan oder Tetrahydrofuran, oder eines Lösungsmittelgemisches, wenn notwendig unter Kühlen, z.B. bei etwa

-30° bis etwa 30°C.

Stufe 4

Das Ausgangsmaterial der Formel III wird erhalten, indem man eine Verbindung der Formel VIII mit einer geeigneten Phosphin-Verbindung, wie einem Triniederalkylphosphin, z.B. Tri-n-butyl-phosphin, oder einem Triarylphosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, und eine gegebenenfalls erhältlichen Verbindung der Formel III′ worin W′ für Triphenylmethylthio oder Niederalkanoylthio steht, in eine Verbindung der Formel III′ (=III) überführt, worin W′ für den Rest

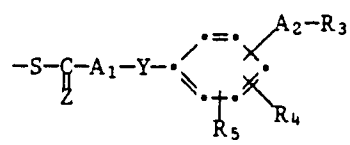

steht.

Die Umsetzung mit der Phosphin- bzw. Phosphit-Verbindung wird vorzugsweise in einem geeigneten inerten Lösungsmittel, wie einem Kohlenwasserstoff, oder einem Aether oder in einem Lösungsmittelgemisch vorgenommen. Je nach Reaktionsfähigkeit arbeitet man unter Kühlen oder bei erhöhter Temperatur, etwa zwischen -10° und +100°C, bevorzugt bei etwa 20° bis 80°C. Ueblicherweise arbeitet man in Gegenwart eines basischen Mittels, wie einer organischen Base, z.B. eines Amins, oder "Polystyol-Hünigbase", oder einer anorganischen Base, z.B. eines Alkalimetallcarbonats, wobei die primär entstandene Phosphoniumverbindung der Formel

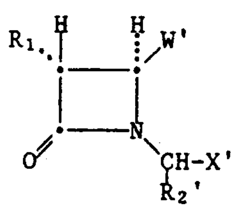

(IIIa),

worin X′ eine Phosphonogruppe oder eine Phosphoniogruppe zusammen mit einem Anion, je nach Bedeutung des Restes $X_o$ (vgl. Formel VIII) z.B. Chlorid, bedeutet, in das Ylid-Ausgangsmaterial der Formel III umgewandelt wird.

Die Einführung des Restes

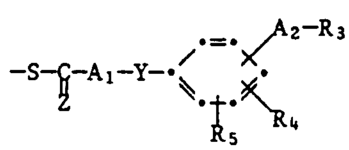

in Verbindungen der Formel III′, worin W′ für Niederalkanoylthio oder Triphenylmethylthio steht, kann in analoger Weise erfolgen, wie unter Stufe 2 beschrieben.

Die Ausgangsverbindungen der Formel II sind bekannt oder können z.B. hergestellt werden, indem man ein Phosphoran der Formel

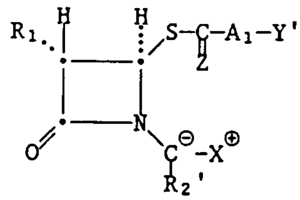

(X),

welches in analoger Weise, wie im Reaktionsschema I gezeigt, synthetisiert werden kann, ringschliesst, z.B. wie unter Verfahren b) beschrieben.

Die für den Aufbau der Seitenkette in Position 2 der erfindungsgemässen Penem-Verbindungen benötigten Ausgangsverbindungen, z.B. diejenigen der Formeln Va, Vb, IXa und IXb sind bekannt oder können in Analogie zu den in der Literatur beschriebenen Verfahren hergestellt werden.

Man kann das im Reaktionsschema I beschriebene Verfahren zur Herstellung der Verbindungen der Formeln (III), (IV), (VII) und (VIII), sowie das angegebene Verfahren zur Herstellung der Verbindungen der Formel (II) und der Endprodukte der Formel (I) auch mit optisch inaktiven Verbindungen durchführen und auf einer beliebigen Verfahrensstufe aus einem erhaltenen Diastereomerengemisch oder Racemat in bekannter Weise die optisch aktiven Verbindungen gemäss vorliegender Erfindung isolieren.

Vorzugsweise werden solche Ausgangsverbindungen verwendet und die Reaktionsbedingungen so gewählt, dass man zu den vorstehend als besonders bevorzugt aufgeführten Verbindungen der Formel I gelangt.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. z.B. intramuskulären, intravenösen, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, enthalten. Tabletten enthalten ebenfalls Bindemittel, z.B. Magnesiumaluminiumsilikat, Stärken, wie Mais-, Weizen-, Reis- oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventioneller Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man Dosen (oral oder parenteral) von etwa 100 mg bis etwa 1000 mg b.i.d. oder t.i.d. zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

Experimenteller Teil

Beispiel 1: (5R,6S)-2-[4-(N-Allyloxycarbonylaminomethyl)-phenoxymethyl]-6-[(1R)-1-allyloxycarbonyloxyäthyl]-2-penem-3-carbonsäure-allylester (Ausgangsstoff)

Eine Lösung von 8,4 g 2-[(3S,4R)-3-[(1R)-1-Allyloxycarbonyloxyäthyl]-4-[4-(N-allyloxycarbonylaminomethyl)-phenoxyacetylthio]-2-oxo-azetidin-1-yl]-2-triphenylphosphoranylidenessigsäureallylester in 750 ml Toluol wird unter Argonatmosphäre 45 Minuten bei Rückflusstemperatur gerührt. Dann wird das Lösungsmittel abgedampft und das Rohprodukt durch Chromatographie an Silicagel gereinigt. $R_f$ (Laufmittel Toluol/Aethylacetat 1:1) = 0,6. IR ($CH_2Cl_2$): 3440; 1793; 1745; 1720; 1590 $cm^{-1}$.

Das Ausgangsmaterial kann wie folgt hergestellt werden:

a. 4-(N-Allyloxycarbonylaminomethyl)-phenoxyessigsäure

0,97 g 4-Aminomethylphenoxyessigsäure werden in 15 ml Wasser suspendiert, mit 2,4 ml 5N NaOH-Lösung und nach Eiskühlung innert 5 min. mit 0,63 ml Chlorameisensäureallylester versetzt. Nach Entfernen des Kühlbades wird das Reaktionsgemisch während 20 min. bei Raumtemperatur gerührt. Das Reaktionsgemisch wird dann zweimal mit Aethylacetat gewaschen, die wässrige Phase wird mit 4N HCl auf pH 2 gestellt und mit Aethylacetat extrahiert. Die organische Lösung wird mit Sole gewaschen und über Natriumsulfat getrocknet. Die Titelverbindung erhält man nach Abdampfen des Lösungsmittels. DC (Aethylacetat/Essigsäure 95:5) $R_f$ = 0,4.

b. 2-[(3S,4R)-3-[(1R)-1-Allyloxycarbonyloxyäthyl]-4-[4-(N-allyloxycarbonylaminomethyl)-phenoxyacetylthio)-2-oxo-azetidin-1-yl]-2-triphenylphosphoranylidenessigsäureallylester

18,5 g Silbersalz des 2-[(3S,4R)-3-[(1R)-1-Allyloxycarbonyloxyäthyl]-4-mercapto-2-oxo-azetidin-1-yl]-2-triphenylphosphoranylidenessigsäureallylesters werden in 320 ml Methylenchlorid gelöst, bei 0° mit 4,36 ml Pyridin, 100 mg 4-Dimethylaminopyridin und anschliessend tropfenweise mit einer Lösung von 12 g 4-(N-Allyloxycarbonylaminomethyl)-phenoxyacetylchorid in 210 ml Methylenchlorid versetzt. Nach 20 min Rühren bei 0° wird der Feststoff über Hyflo abfiltriert, und das Filtrat wird mit wässriger Natriumhydrogencarbonat-Lösung und dann mit gesättigter NaCl-Lösung gewaschen. Nach Trocknen über $Na_2SO_4$ wird das Lösungsmittel abgedampft. Der Rückstand wird durch Chromatographie an Silicagel gereinigt (Laufmittel Toluol bis Toluol/Aethylacetat 75:25) $R_f$ =0,33. IR ($CH_2Cl_2$): 3440; 1760; 1720; 1695 $cm^{-1}$.

Beispiel 2: (5R,6S)-2-[4-(Aminomethyl)-phenoxymethyl]-6-[(1R)-1-hydroxyäthyl]-2-penem-3-carbonsäure

Eine Lösung von 0,528 g (5R,6S)-2-[4-(N-Allyloxycarbonylaminomethyl)-phenoxymethyl]-6-[(1R)-1-allyloxycarbonyloxyäthyl]-2-penem3-carbonsäureallylester in 14 ml absolutem THF wird portionsweise mit 43 mg Tetrakis-triphenylphosphin-palladium und 1,51 ml Tributylzinnhydrid versetzt. Nach 1,5 h Rühren bei Raumtemperatur wird das Gemisch tropfenweise mit 0,32 ml Essigsäure versetzt und weitere 15 min gerührt. Das Reaktionsgemisch wird mit 15 ml Hexan versetzt. Nach Zentrifugieren und Abdekantieren der Flüssigkeit wird der Rückstand, nach erneutem Waschen mit Hexan, mit Wasser verrieben und abfiltriert. Das Nutschgut wird noch fünfmal mit ca. 10 ml Wasser gewaschen. Das gesammelte Filtrat wird am Rotationsverdampfer konzentriert, und durch Chromatographie an OPTI $UPC_{12}$ (Laufmittel: Wasser) erhält man die Titelverbindung. DC (OPTI $UPC_{12}$; Wasser) $R_f$ = 0,5. IR (DMSO-$d_6$): 3445; 2970; 1774; 1613; 1583 $cm^{-1}$.
UV (Wasser): $\lambda_{max}$ = 307 nm.

Beispiel 3: (5R,6S)-Pivaloyloxymethyl-2-(4-fluorphenoxymethyl)-6-[(1R)-1-hydroxyäthyl]-2-penem-3-carboxylat (Analogiebeispiel: siehe Beispiel 8)

90 mg (5R,6S)-2-(4-Fluorphenoxymethyl)-6-[(1R)-1-hydroxyäthyl]-2-penem-3-carbonsäure, Natriumsalz werden in 2 ml abs. DMF gelöst, auf 0° gekühlt und mit 67 µl Jodmethylpivalat versetzt. Nach 45 min Rühren bei 0° wird mit Aethylacetat verdünnt, dreimal mit gesättigter wässriger Kochsalz-Lösung gewaschen und über $Na_2SO_4$ getrocknet. Nach Filtrieren und Einengen wird das rohe Produkt durch Chromatographie an Silicagel (Lösungsmittel: Toluol bis Toluol/Aethylacetat 85:15) gereinigt. IR($CH_2Cl_2$): 1795, 1750, 1730, 1585 $cm^{-1}$,
UV(Aethanol): $\lambda_{max}$ = 328 nm.

Beispiel 4: (5R,6S)-2-(4-Allyloxycarbonylaminophenoxymethyl]-6-[(1R)-1-allyloxycarbonyloxyäthyl]-2-penem-3-carbonsäureallylester (Ausgangsstoff)

Analog Beispiel 1 werden 1,15 g 2-[(3S,4R)-3-[(1R)-1-Allyloxycarbonyloxyäthyl]-4-(4-allyloxycarbonylaminophenoxyacetylthio)-2-oxo-azetidin-1-yl]-2-triphenylphosphoranylidenessigsäureallylester in 150 ml Toluol zur Titelverbindung umgesetzt. IR($CH_2Cl_2$): 3420, 1792, 1735, 1710, 1645 $cm^{-1}$
Das Ausgangsmaterial kann wie folgt hergestellt werden:

a. 4-(Allyloxycarbonylamino)-phenoxyessigsäure

0,9 g 4-Aminophenoxyessigsäure werden in 15 ml Wasser suspendiert, mit 2,4 ml 5N NaOH-Lösung und nach Eiskühlung innert 5 min mit 0,63 ml Chlorameisensäureallylester versetzt. Nach Entfernen des Kühlbades wird das Reaktionsgemisch während 20 min bei Raumtemperatur gerührt. Das Reaktionsgemisch wird dann zweimal mit Aethylacetat gewaschen, die wässrige Phase wird mit 4N HCl auf pH 2 gestellt und mit Aethylacetat extrahiert. Die organische Lösung wird mit Sole gewaschen, über Natriumsulfat getrocknet. Die Titelverbindung erhält man nach Abdampfen des Lösungsmittels. DC (Aethylacetat/Essigsäure 95:5) $R_f$ = 0,4.

b. 2-[(3S,4R)-3-[(1R)-1-Allyloxycarbonyloxyäthyl]-4-(4-allyloxycarbonylaminophenoxyacetylthio)-2-oxo-azetidin-1-yl]-2-triphenylphosphoranylidenessigsäureallylester.

1 g 4-(Allyloxycarbonylamino)-phenoxyessigsäure wird in 2 ml $CH_2Cl_2$ suspendiert und mit 0,6 ml 1-Chlor-1-dimethylamino-2-methyl-prop-1-en versetzt. Man lässt 75 min rühren und lässt das Gemisch danach zu einer Lösung von 1,74 g Silbersalz des 2-[(3S,4R)-3-[(1R)-1-Allyloxycarbonyloxyäthyl]-4-mercapto-2-oxo-azetidin-1-yl]-2-triphenylphosphoranylidenessigsäureallylesters, 50 mg Dimethylaminopyridin und 0,41 ml Pyridin in 30 ml absolutem Methylenchlorid innert 5 min zutropfen. Nach dem Aufarbeiten analog Beispiel 1 erhält man die Titelverbindung. IR($CH_2Cl_2$): 3420, 1755, 1730, 1690 $cm^{-1}$.

Beispiel 5: (5R,6S)-2-(4-Aminophenoxymethyl)-6-[(1R)-1-hydroxyäthyl]-2-penem-3-carbonsäure, Natriumsalz

530 mg (5R,6S)-2-(4-Allyloxycarbonylaminophenoxymethyl)-6-[(1R)-1-allyloxycarbonyloxyäthyl]-2-penem-3-carbonsäureallylester werden in 16 ml abs. THF gelöst und unter Rühren nacheinander mit 40 mg Tetrakis-triphenylphosphin-palladium und 1,1 ml Tributylzinnhydrid versetzt. Nach 30 min gibt man nochmals 20 mg Tetrakis-triphenylphosphin-palladium und 0,32 ml Tributylzinnhydrid dazu und lässt weitere 1,5 Stunden rühren. 15 Minuten nach Zugabe von 0,31 ml Essigsäure wird das Reaktionsgemisch im Vakuum eingedampft und in Wasser/Aethylacetat aufgetrennt. Der pH-Wert wird mittels Natriumhydrogencarbonat auf 7,7 eingestellt. Nach zweimaligem Waschen mit Aethylacetat wird die wässrige Lösung im Vakuum konzentriert. Die reine Titelverbindung wird durch Säulenchromatographie an OPTI $UPC_{12}$ (Lösungmittel: Wasser) erhalten. IR(DMSO-$d_6$): 1771, 1616, 1587, 1511 $cm^{-1}$, UV(Wasser) $\lambda_{max}$ = 302 nm

Beispiel 6: (5R,6S)-2-[4-(2-Allyloxycarbonylaminoäthyl)-phenoxymethyl]-6-[(1R)-1-allyloxycarbonyloxy-äthyl]-2-penem-3-carbonsäureallylester (Ausgangsstoff)

Analog Beispiel 1 werden 2 g 2-[(3S,4R)-3-[(1R)-1-Allyloxycarbonyloxyäthyl]-4-(4-(2-allyloxycarbonylaminoäthyl)-phenoxyacetylthio)-2-oxo-azetidin-1-yl]-2-triphenylphosphoranylidenessigsäureallylester in 400 ml abs. Toluol in die Titelverbindung überführt. IR($CH_2Cl_2$): 3430, 1790, 1740, 1710, 1640 $cm^{-1}$.

Die Ausgangsverbindung kann wie folgt hergestellt werden:

a. 4-(2-Allyloxycarbonylaminoäthyl)-phenoxyessigsäure

Analog Beispiel 4 werden 3,9 g 4-(2-Aminoäthyl)-phenoxyessigsäure in die Titelverbindung überführt. DC (Aethylacetat/Essigsäure 95:5) $R_f$ = 0,4.

b. 2-[(3S,4R)-3-[(1R)-1-Allyloxycarbonyloxyäthyl]-4-(4-(2-allyloxycarbonylaminoäthyl)-phenoxyacetylthio)-2-oxo-azetidin-1-yl]-2-triphenylphosphoranylidenessigsäureallylester

Analog Beispiel 4 werden 2,25 g 4-(2-Allyloxycarbonylaminoäthyl)phenoxyessigsäure mit 3,48 g Silbersalz des 2-[(3S,4R)-3-[(1R)-1-Allyloxycarbonyloxyäthyl]-4-mercapto-2-oxo-azetidin-1-yl]-2-triphenylphosphoranylidenessigsäureallylesters in die Titelverbindung überführt. IR ($CH_2Cl_2$): 3430, 1750, 1715, 1690 $cm^{-1}$

Beispiel 7: (5R,6S)-2-(4-(2-Aminoäthyl)-phenoxymethyl]-6-[(1R)-1-hydroxyäthyl]-2-penem-3-carbonsäure

Analog Beispiel 5 werden 0,97 g (5R,6S)-2-[4-(2-Allyloxycarbonylaminoäthyl)-phenoxymethyl]-6-[(1R)-1-allyloxycarbonyloxyäthyl]-2-penem-3-carbonsäureallylester mit Tributylzinnhydrid und Tetrakistriphenylphosphin-palladium in die Titelverbindung überführt. IR(DMSO-$d_6$): 1774, 1613, 1585, 1512 $cm^{-1}$, UV (Wasser):
$\lambda_{max}$ = 307 nm.

Beispiel 8: Pivaloyloxymethyl-(5R,6S)-2-phenoxymethyl-6-[(1R)-1-hydroxyäthyl]-2-penem-3-carboxylat

Analog Beispiel 3 werden 103 mg Natrium (5R,6S)-2-phenoxymethyl-6-[(1R)-1-hydroxyäthyl]-2-penem-3-carboxylat mit 80 µl Jodmethylpivalat in 2,2 ml abs. DMF in die Titelverbindung überführt. IR ($CH_2Cl_2$): 3600, 1790, 1750, 1725, 1597, 1587 $cm^{-1}$,
UV (Aethanol) $\lambda_{max}$ = 327 nm.

Beispiel 9: (5R,6S)-2-[4-(1-Methyltetrazol-5-ylthiomethyl)-phenoxymethyl]-6-[(1R)-1-allyloxycarbonyloxyäthyl]-2-penem-3-carbonsäure-allylester (Analogiebeispiel: siehe Beispiele 10 und 13).

Eine Lösung von 400 mg (5R,6S)-2-(4-Chlormethylphenoxymethyl)-6-[(1R)-1-allyloxycarbonyloxyäthyl]-2-penem-3-carbonsäureallylester in 2 ml abs. DMF wird auf 0° gekühlt und nacheinander mit 0,64 g pulverisiertem Kaliumjodid und einer Lösung von 132 mg Natrium-1-methyltetrazol-5-thiolat in 0,8 ml DMF versetzt. Man belässt die Reaktionsmischung während 15 min bei 0° und dann 30 min bei Raumtemperatur. Danach wird das DMF am Hockvakuum abgezogen, und der Rückstand wird zwischen Wasser und Aethylacetat aufgeteilt. Die organische Phase wird abgetrennt, mit Sole gewaschen, über Natriumsulfat getrocknet und eingeengt. Nach Reinigung durch Chromatographie an Silicagel (Laufmittel Toluol bis Toluol/Aethylacetat 92,5:7,5) erhält man die reine Titelverbindung.
IR ($CH_2Cl_2$): 1795, 1750, 1710, 1650, 1610, 1590 $cm^{-1}$

Beispiel 10: (5R,6S)-2-[4-(4-Morpholinomethyl)-phenoxymethyl]-6-[(1R)-1-allyloxycarbonyloxyäthyl]-2-penem-3-carbonsaureallylester (Ausgangsstoff)

Analog Beispiel 9 werden 0,5 g (5R,6S)-2-(4-Chlormethylphenoxymethyl)-6-[(1R)-1-allyloxycarbonyloxyäthyl]-2-penem-3-carbonsäureallylester mit 3,3 g Kaliumjodid und 105 µl Morpholin in 10 ml abs. DMF in die Titelverbindung überführt.
IR ($CH_2Cl_2$): 1790, 1750, 1705, 1650, 1610, 1590 $cm^{-1}$

Beispiel 11: (5R,6S)-2-[4-(4-Morpholinomethyl)-phenoxymethyl]-6-[(1R)-1-hydroxyäthyl]-2-penem-3-carbonsäure, Natriumsalz

Analog Beispiel 2 werden 455 mg (5R,6S)-2-[4-(4-Morpholinomethyl)phenoxymethyl]-6-[(1R)-1-allyloxycarbonyloxyäthyl]-2-penem-3-carbonsäureallylester mit 24 mg Tetrakis-triphenylphosphin-palladium und 131 mg 1,3-Dimethylbarbitursäure in 9 ml THF in die Titelverbindung überführt.
IR (DMSO-$d_6$): 1775, 1612, 1585, 1510 $cm^{-1}$.
UV (Wasser): $\lambda_{max}$ = 307 nm

Beispiel 12: In analoger Weise, wie in den vorangehenden Beispielen beschrieben, werden die folgenden Verbindungen hergestellt:

(5R,6S)-2-[3-(Aminomethyl)-phenoxymethyl]-6-[(1R)-1-hydroxyäthyl]-2-penem-3-carbonsäure

IR (DMSO-$D_6$): 3435; 2968; 1774; 1616; 1587 $cm^{-1}$,
UV (Wasser) $\lambda_{max}$ = 308 nm

(5R,6S)-2-[2-(Aminomethyl)-phenoxymethyl]-6-[(1R)-1-hydroxyäthyl]-2-penem-3-carbonsäure

IR (DMSO-$D_6$): 3436; 2968; 1774; 1617; 1585 $cm^{-1}$
UV (Wasser) $\lambda_{max}$ = 307 nm

(5R,6S)-2-[2-Aminophenoxymethyl]-6-[(1R)-1-hydroxyäthyl]-2-penem-3-carbonsäure

UV (Wasser) $\lambda_{max}$ = 306 nm

(5R,6S)-2-[4-Carbamoylphenoxymethyl]-6-[(1R)-1-hydroxyäthyl]-2-penem-3-carbonsäure

UV (Wasser) $\lambda_{max}$ = 300 nm

(5A,6S)-2-[2-Carbamoylphenoxymethyl]-6-[(1R)-1-hydroxyäthyl]-2-penem-3-carbonsäure

UV (Wasser) $\lambda_{max}$ = 299 nm
IR (DMSO-$d_6$): 3453; 1773; 1666; 1620; 1595 $cm^{-1}$

Beispiel 13: In analoger Weise, wie in den vorangehenden Beispielen beschrieben, werden die folgenden Verbindungen hergestellt (Verfahren A: vgl. Beispiel 1; Verfahren B: vgl. Beispiel 9):

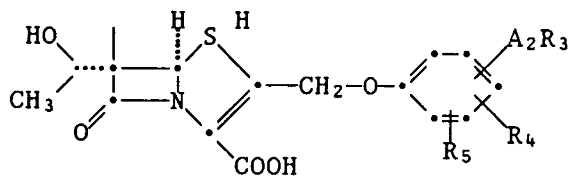

| $A_2$-$R_3$ | UV (Wasser) $\lambda_{max}$ (nm) | IR (DMSO-$d_6$) ($cm^{-1}$) | Verfahren |
|---|---|---|---|
| 4-$CH_2NHCH_3$ | 306 | 3435,2968,1773, 1615,1586 | A |
| 3-$CH_2NHCH_3$ | 305 | | A |
| 2-$CH_2NHCH_3$ | 306 | | A |
| 4-$CH_2NHCH_2CH_2OH$ | 307 | | A |
| 3-$CH_2NHCH_2CH_2OH$ | 306 | | A |
| 2-$CH_2NHCH_2CH_2OH$ | 306 | | A |
| 4-$CH_2$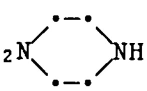 | 306 | | B |
| 4-$CH_2$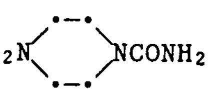 | 306 | | B |
| 4-$CH_2NH$-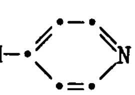 | 308 | | A |
| 4-$CH_2NH$-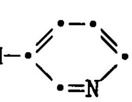 | 307 | | A |

| $A_2$-$R_3$ | UV (Wasser) $\lambda_{max}$ (nm) | Verfahren |
|---|---|---|
| 4-$CH_2N(CH_2CH_2OH)_2$ | 305 | B |
| 4-$CH_2$ 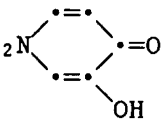 | 310 | B |
| 4-$CH_2S$- 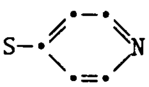 | 308 | B |
| 4-$CH_2$- 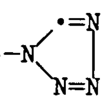 | 306 | A |

Wenn nicht anders angegeben, stehen $R_4$ und $R_5$ für Wasserstoff.

Beispiel 14: (5R,6S)-2-[4-(Dimethylaminomethyl)-phenoxymethyl]-6-[(1R)-1-hydroxyäthyl]-2-penem-3-carbonsäure, Natriumsalz

70 mg (5R,6S)-2-[4-(Aminomethyl)-phenoxymethyl]-6-[(1R)-1-hydroxyäthyl]-2-penem-3-carbonsäure werden in 7 ml wässrigem Tetrahydrofuran gelöst. Nach Einstellen auf pH 6.81 mittels 1N HCl wird die Lösung mit 50 µl einer 37 % wässrigen Formaldehyd-Lösung versetzt und 10 Minuten bei Raumtemperatur unter Stickstoff gerührt (ph ~5.9). Nach Zugabe eines Gemisches aus 12,5 mg Natriumcyanoborhydrid und 13,75 mg Zinkchlorid in 1,5 ml Puffer pH 7 wird noch mit Natriumhydrogencarbonat pH 7.1 eingestellt. Nach 45 Minuten Rühren bei Raumtemperatur wird das Reaktionsgemisch unter vermindertem Druck am Rotationsverdampfer konzentriert und der gelbe Rückstand wird in Wasser auf die Chromatographiesäule aufgetragen. (OPTI UPC12; Wasser, dann Wasser/Acetonitril 9:1). Man erhält so die gewünschte Titelverbindung.
DC(OPTI UPC12; Wasser/Acetonitril 4:1), $R_f$ = 0,29.
UV($H_2O$): $\lambda_{max}$ = 307 nm.

Beispiel 15: Trockenampullen oder Vials, enthaltend 0,5 g (5R,6S)-[4-(Aminomethyl)-phenoxymethyl]-6-[-(1R)-1-hydroxyäthyl]-2-penem-3-carbonsäure als Wirksubstanz, werden wie folgt hergestellt:

Zusammensetzung (für 1 Ampulle oder Vial):

| | |
|---|---|
| Wirksubstanz | 0,5 g |
| Mannit | 0,5 g |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann auch dieselbe Menge eines anderen Wirkstoffs der vorangehenden Beispiele verwendet werden.

**Patentansprüche**
**Patentansprüche für folgende Vertragsstaaten : AT, BE, CH, DE, FR, GB, IT, LI, LU, NL, SE**

1. Verbindungen der Formel

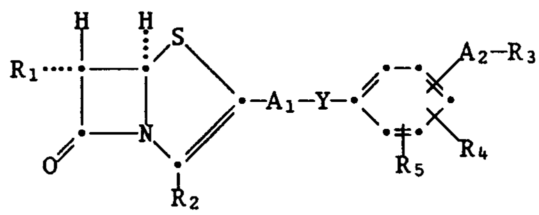

(I),

worin $R_1$ Hydroxymethyl oder 1-Hydroxyäthyl ist, $R_2$ Carboxy oder unter physiologischen Bedingungen spaltbares verestertes Carboxy bedeutet, $R_3$ Amino, Methylamino, Dimethylamino, (2-Hydroxyäthyl)-amino, Bis-(2-hydroxyäthyl)-amino, (4-Pyridyl)-amino, (3-Pyridyl)-amino, Morpholin-4-yl, Piperazin-1-yl, 4-Carbamoyl-piperazin-1-yl, 3-Hydroxy-4-oxo-1,4-dihydro-pyridin-1-yl, 1H-Tetrazol-1-yl, Pyridin-4-ylthio oder Carbamoyl ist, $R_4$ und $R_5$ Wasserstoff bedeuten, Y für eine Gruppe -O- steht, $A_1$ $C_1$-$C_2$-Alkylen ist und $A_2$ für eine direkte Bindung steht oder $C_1$-$C_2$-Alkylen bedeutet, die reinen optischen Isomere von Verbindungen der Formel I und Mischungen dieser optischen Isomere, und Salze von Verbindungen der Formel I, die eine salzbildende Gruppe aufweisen, mit Ausnahme von quaternären Ammoniumsalzen.

2. Verbindungen der Formel I gemäss Anspruch 1, worin $R_1$ Hydroxymethyl oder 1-Hydroxyäthyl ist, $R_2$ Carboxy oder unter physiologischen Bedingungen spaltbares verestertes Carboxy ist, $R_3$ Amino oder Carbamoyl bedeutet, $R_4$ und $R_5$ jeweils Wasserstoff bedeuten, Y für eine Gruppe -O- steht, $A_1$ $C_1$-$C_2$-Alkylen ist und $A_2$ für eine direkte Bindung steht oder $C_1$-$C_2$-Alkylen bedeutet, die reinen optischen Isomere von Verbindungen der Formel I und Mischungen dieser optischen Isomere, und Salze davon mit Ausnahme von quaternären Ammoniumsalzen.

3. Verbindungen der Formel I gemäss Anspruch 1, worin $R_1$ 1-Hydroxyäthylist, $R_2$ Carboxy ist, $A_3$ Amino, Methylamino oder Dimethylamino bedeutet, $R_4$ und $R_5$ jeweils Wasserstoff bedeuten, Y für eine Gruppe -O- steht, $A_1$ Methylen ist und $A_2$ Methylen oder Aethylen ist, die reinen optischen Isomere von Verbindungen der Formel I und Mischungen dieser optischen Isomere, und Salze davon mit Ausnahme von quaternären Ammoniumsalzen.

4. Pharmazeutisch annehmbare Salze von Verbindungen der Formel I gemäss Anspruch 1.

5. (5R,6S)-2-[4-(Aminomethyl)-phenoxymethyl]-6-[(1R)-1-hydroxyäthyl]-2-penem-3-carbonsäure und pharmazeutisch annehmbare Salze davon gemäss Anspruch 1.

6. (5R,6S)-2-[4-Aminophenoxymethyl]-6-[(1R)-1-hydroxyäthyl]-2-penem-3-carbonsäure und pharmazeutisch annehmbare Salze davon gemäss Anspruch 1.

7. (5R,6S)-2-[4-(4-Morpholinomethyl)-phenoxymethyl]-6-[(1R)-1-hydroxyäthyl]-2-penem-3-carbonsäure und pharmazeutisch annehmbare Salze davon gemäss Anspruch 1.

8. (5R,6S)-2-[4-(Dimethylaminomethyl)-phenoxymethyl]-6-[(1R)-1-hydroxyäthyl]-2-penem-3-carbonsäure und pharmazeutisch annehmbare Salze davon gemäss Anspruch 1.

9. (5R,6S)-2-[4-(2-Aminoäthyl)-phenoxymethyl]-6-[(1R)-1-hydroxyäthyl]-2-penem-3-carbonsäure und pharmazeutisch annehmbare Salze davon gemäss Anspruch 1.

10. Eine Verbindung gemäss Anspruch 1 zur Anwendung in einem Verfahren zur therapeutischen Behandlung des menschlichen und tierischen Körpers.

**11.** Pharmazeutisches Präparat enthaltend eine Verbindung der Formel I oder ein pharmazeutisch annehmbares Salz davon gemäss Anspruch 1, zusammen mit pharmazeutisch annehmbaren Trägerstoffen.

**12.** Verwendung von Verbindungen der Formel I oder von pharmazeutisch annehmbaren Salzen davon gemäss Anspruch 1 zur Herstellung von pharmazeutischen Präparaten zur Anwendung als antibakterielle Antibiotika zur Behandlung von Infektionen.

**13.** Verfahren zur Herstellung von Verbindungen der Formel I und von Salzen davon gemäss Anspruch 1, dadurch gekennzeichnet, dass man

a) in einer Verbindung der Formel

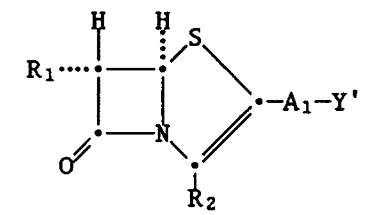

(II),

worin $R_1$, $R_2$ und $A_1$ die unter Formel (I) angegebenen Bedeutungen haben, und Y' eine in den Rest

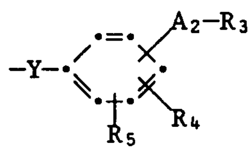

(IIa),

worin Y, $A_2$, $R_3$, $R_4$ und $R_5$ die unter Formel (I) angegebenen Bedeutungen haben, überführbare Gruppe ist, die Gruppe Y' in den Rest

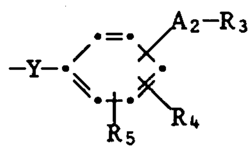

(IIa)

überführt, oder

b) eine Ylid-Verbindung der Formel

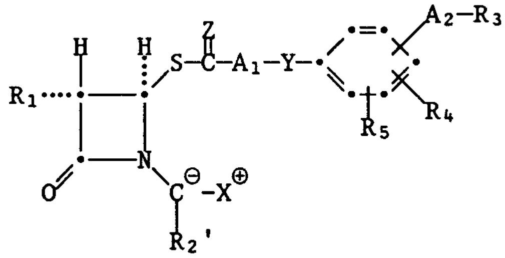

(III),

worin $R_1$, $R_3$, $R_4$, $R_5$, $A_1$, $A_2$ und Y die unter Formel I angegebenen Bedeutungen haben, $R_2$' eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

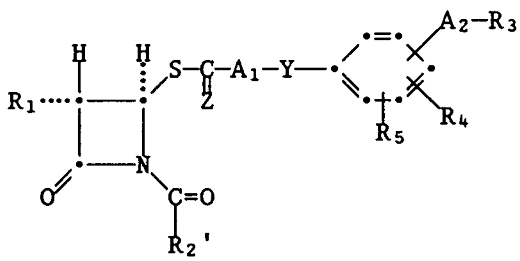

(IV),

worin $R_1$, $R_3$, $R_4$, $R_5$, $A_1$, $A_2$ und Y die unter Formel I angegebenen Bedeutungen haben, Z Sauerstoff oder Schwefel ist und $R_2$' eine geschützte Carboxylgruppe ist, mit einer organischen Verbindung des dreiwertigen Phosphors behandelt, und wenn notwendig, in einer erhältlichen Verbindung der Formel I eine geschützte funktionelle Gruppen in die freie funktionelle Gruppe überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

**14.** Die nach dem Verfahren gemäss Anspruch 13 erhältlichen Verbindungen.

**Patentansprüche für folgende Vertragsstaaten : ES, GR**

**1.** Verfahren zur Herstellung von Verbindungen der Formel

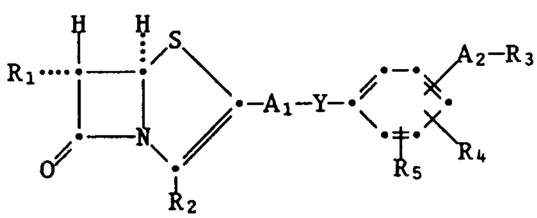

(I),

worin $R_1$ Hydroxymethyl oder 1-Hydroxyäthyl ist, $R_2$ Carboxy oder unter physiologischen Bedingungen spaltbares verestertes Carboxy bedeutet, $R_3$ Amino, Methylamino, Dimethylamino, (2-Hydroxyäthyl)-amino, Bis-(2-hydroxyäthyl)-amino, (4-Pyridyl)-amino, (3-Pyridyl)-amino, Morpholin-4-yl, Piperazin-1-yl, 4-Carbamoyl-piperazin-1-yl, 3-Hydroxy-4-oxo-1,4-dihydro-pyridin-1-yl, 1H-Tetrazol-1-yl, Pyridin-4-ylthio, oder Carbamoyl ist, $R_4$ und $R_5$ Wasserstoff bedeuten, Y für eine Gruppe -O- steht, $A_1$ $C_1$-$C_2$-Alkylen ist und $A_2$ für eine direkte Bindung steht oder $C_1$-$C_2$-Alkylen bedeutet, den reinen optischen Isomeren von Verbindungen der Formel I und Mischungen dieser optischen Isomere, und Salzen von Verbindungen der Formel I, die eine salzbildende Gruppe aufweisen, mit Ausnahme von quaternären Ammoniumsalzen, dadurch gekennzeichnet, dass man

a) in einer Verbindung der Formel

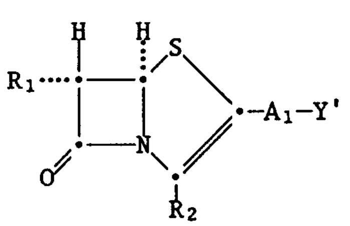

(II),

worin $R_1$, $R_2$ und $A_1$ die unter Formel (I) angegebenen Bedeutungen haben, und Y' eine in den Rest

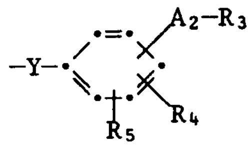

(IIa),

worin Y, $A_2$, $R_3$, $R_4$ und $R_5$ die unter Formel (I) angegebenen Bedeutungen haben, überführbare Gruppe ist, die Gruppe Y' in den Rest

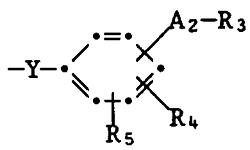

(IIa)

überführt, oder

b) eine Ylid-Verbindung der Formel

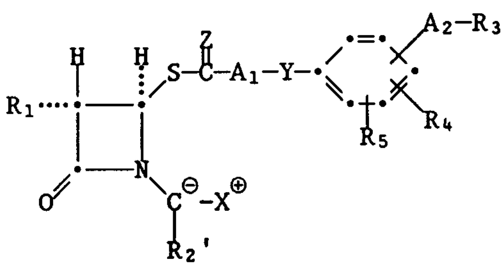

(III),

worin $R_1$, $R_3$, $R_4$, $R_5$, $A_1$, $A_2$ und Y die unter Formel I angegebenen Bedeutungen haben, $R_2$' eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

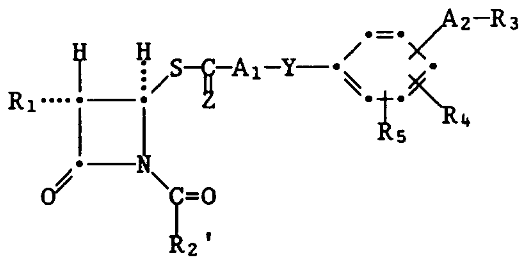

(IV),

worin $R_1$, $R_3$, $R_4$, $R_5$, $A_1$, $A_2$ und Y die unter Formel I angegebenen Bedeutungen haben, Z Sauerstoff oder Schwefel ist und $R_2$' eine geschützte Carboxylgruppe ist, mit einer organischen Verbindung des dreiwertigen Phosphors behandelt, und wenn notwendig, in einer erhältlichen Verbindung der Formel I eine geschützte funktionelle Gruppen in die freie funktionelle Gruppe überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, ein erhältliches Gemisch von isomeren Verbindungen der Formel I in die einzelnen Isomere auftrennt, und/oder, wenn erwünscht, eine

erhältliche Verbindung mit salzbildender Gruppe in ein Salz oder ein erhältliches Salz in die freie Verbindung oder in ein anderes Salz überführt.

2. Verfahren zur Herstellung von Verbindungen der Formel I gemäss Anspruch 1, worin $R_1$ Hydroxymethyl oder 1-Hydroxyäthyl ist, $R_2$ Carboxy oder unter physiologischen Bedingungen spaltbares verestertes Carboxy ist, $R_3$ Amino oder Carbamoyl bedeutet, $R_4$ und $R_5$ jeweils Wasserstoff bedeuten, Y für eine Gruppe -O- steht, $A_1$ $C_1$-$C_2$-Alkylen ist und $A_2$ für eine direkte Bindung steht oder $C_1$-$C_2$-Alkylen bedeutet, der reinen optischen Isomeren von Verbindungen der Formel I und Mischungen dieser optischen Isomere, und Salzen davon mit Ausnahme von quaternären Ammoniumsalzen.

3. Verfahren zur Herstellung von Verbindungen der Formel I gemäss Anspruch 1, worin $R_1$ 1-Hydroxyäthyl ist, $R_2$ Carboxy ist, $R_3$ Amino, Methylamino oder Dimethylamino bedeutet, $R_4$ und $R_5$ jeweils Wasserstoff bedeuten, Y für eine Gruppe -O- steht, $A_1$ Methylen ist und $A_2$ Methylen oder Aethylen ist, der reinen optischen Isomeren von Verbindungen der Formel I und Mischungen dieser optischen Isomere, und Salzen davon mit Ausnahme von quaternären Ammoniumsalzen.

4. Verfahren zur Herstellung von pharmazeutisch annehmbaren Salzen von Verbindungen der Formel I gemäs Anspruch 1.

5. Verfahren gemäss Anspruch 1 zur Herstellung von (5R,6S)-2-[4-(Aminomethyl)-phenoxymethyl]-6-[(1R)-1-hydroxyäthyl]-2-penem-3-carbonsäure und pharmazeutisch annehmbaren Salzen davon.

6. Verfahren gemäss Anspruch 1 zur Herstellung von (5R,6S)-2-[4-Aminophenoxymethyl]-6-[(1R)-1-hydroxyäthyl]-2-penem-3-carbonsäure und pharmazeutisch annehmbaren Salzen davon.

7. Verfahren gemäss Anspruch 1 zur Herstellung von (5R,6S)-2-[4-(4-Morpholinomethyl)-phenoxymethyl]-6-[(1R)-1-hydroxyäthyl]-2-penem-3-carbonsäure und pharmazeutisch annehmbaren Salzen davon.

8. Verfahren gemäss Anspruch 1 zur Herstellung von (5R,6S)-2-[4-(Dimethylaminomethyl)-phenoxymethyl]-6-[(1R)-1-hydroxyäthyl]-2-penem-3-carbonsäure und pharmazeutisch annehmbaren Salzen davon.

9. Verfahren gemäss Anspruch 1 zur Herstellung von (5R,6S)-2-[4-(2-Aminoäthyl)-phenoxymethyl]-6-[(1R)-1-hydroxyäthyl]-2-penem-3-carbonsäure und pharmazeutisch annehmbaren Salzen davon.

## Claims

**Claims for the following Contracting States : AT, BE, CH, DE, FR GB IT LI LU NL SE**

1. A compound of the formula

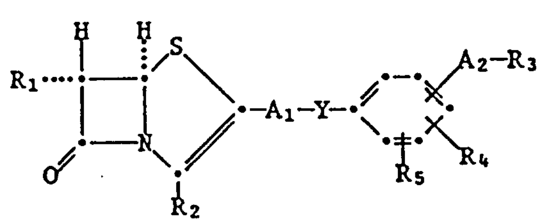

(I)

wherein $R_1$ is hydroxymethyl or 1-hydroxyethyl, $R_2$ is carboxy or esterified carboxy that is cleavable under physiological conditions, $R_3$ is amino, methylamino, dimethylamino, (2-hydroxyethyl)-amino, bis-(2-hydroxyethyl)-amino, (4-pyridyl)-amino, (3-pyridyl)-amino, morpholin-4-yl, piperazin-1-yl, 4-carbamoylpiperazin-1-yl, 3-hydroxy-4-oxo-1,4-dihydropyridin-1-yl, 1H-tetrazol-1-yl, pyridin-4-ylthio or carbamoyl, $R_4$ and $R_5$ are hydrogen, Y is a group -O-, $A_1$ is $C_1$-$C_2$alkylene, and $A_2$ is a direct bond or $C_1$-$C_2$alkylene, a pure optical isomer of a compound of formula I or a mixture of those optical isomers, or a salt of a compound of formula I having a salt-forming group, with the exception of a quaternary ammonium salt.

2. A compound of formula I according to claim 1, wherein $R_1$ is hydroxymethyl or 1-hydroxyethyl, $R_2$ is carboxy or esterified carboxy that is cleavable under physiological conditions, $R_3$ is amino or carbamoyl, each of $R_4$ and $R_5$ is hydrogen, Y is a group -O-, $A_1$ is $C_1$-$C_2$alkylene, and $A_2$ is a direct bond or $C_1$-$C_2$alkylene, a pure optical isomer of a compound of formula I or a mixture of those optical isomers, or a salt thereof, with the exception of a quaternary ammonium salt.

3. A compound of formula I according to claim 1, wherein $R_1$ is 1-hydroxyethyl, $R_2$ is carboxy, $R_3$ is amino, methylamino or dimethylamino, each of $R_4$ and $R_5$ is hydrogen, Y is a group -O-, $A_1$ is methylene, and $A_2$ is methylene or ethylene, a pure optical isomer of a compound of formula I or a mixture of those optical isomers, or a salt thereof, with the exception of a quaternary ammonium salt.

4. A pharmaceutically acceptable salt of a compound of formula I according to claim 1.

5. (5R,6S)-2-[4-(aminomethyl)-phenoxymethyl]-6-[(1R)-1-hydroxyethyl]-2-penem-3-carboxylic acid or a pharmaceutically acceptable salt thereof according to claim 1.

6. (5R,6S)-2-[4-aminophenoxymethyl]-6-[(1R)-1-hydroxyethyl]-2-penem-3-carboxylic acid or a pharmaceutically acceptable salt thereof according to claim 1.

7. (5R,6S)-2-[4-(4-morpholinomethyl)-phenoxymethyl]-6-[(1R)-1-hydroxyethyl]-2-penem-3-carboxylic acid or a pharmaceutically acceptable salt thereof according to claim 1.

8. (5R,6S)-2-[4-(dimethylaminomethyl)-phenoxymethyl]-6-[(1R)-1-hydroxyethyl]-2-penem-3-carboxylic acid or a pharmaceutically acceptable salt thereof according to claim 1.

9. (5R,6S)-2-[4-(2-aminoethyl)-phenoxymethyl]-6-[(1R)-1-hydroxyethyl]-2-penem-3-carboxylic acid or a pharmaceutically acceptable salt thereof according to claim 1.

10. A compound according to claim 1 for use in a method for the therapeutic treatment of the human or animal body.

11. A pharmaceutical composition comprising a compound of formula I or a pharmaceutically acceptable salt thereof according to claim 1, together with pharmaceutically acceptable carriers.

12. The use of a compound of formula I or of a pharmaceutically acceptable salt thereof according to claim 1 for the preparation of a pharmaceutical composition for use as an antibacterial antibiotic for the treatment of infections.

13. A process for the preparation of a compound of formula I or of a salt thereof according to claim 1, wherein

a) in a compound of the formula

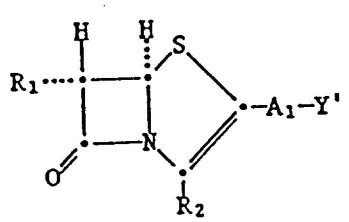

(II),

wherein $R_1$, $R_2$ and $A_1$ are as defined for formula (I) and Y' is a group that can be converted into the radical

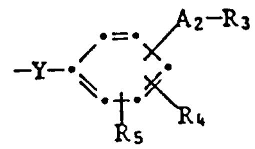

(IIa),

wherein Y, $A_2$, $R_3$, $R_4$ and $R_5$ are as defined for formula (I), the group Y' is converted into the radical

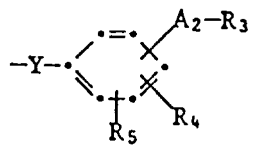

(IIa)

or
b) an ylid compound of the formula

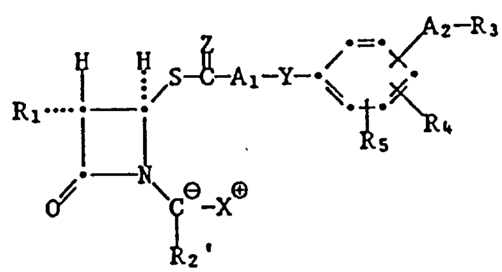

(III),

wherein $R_1$, $R_3$, $R_4$, $R_5$, $A_1$, $A_2$ and Y are as defined for formula I, $R_2$' is a protected carboxy group, Z is oxygen or sulfur and $X^{\oplus}$ is either a trisubstituted phosphonio group or a diesterified phosphono group together with a cation, is cyclised, or
c) a compound of the formula

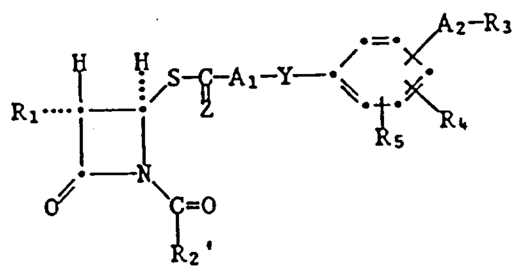

(IV),

wherein $R_1$, $R_3$, $R_4$, $R_5$, $A_1$, $A_2$ and Y are as defined for formula I, Z is oxygen or sulfur and $R_2$' is a protected carboxy group, is treated with an organic compound of trivalent phosphorus, and, if necessary, in an obtainable compound of formula I a protected functional group is converted into the free functional group, and/or, if desired, in an obtainable compound of formula I a free carboxy group $R_2$ is converted into an esterified carboxy group that is cleavable under physiological conditions, and/or, if desired, in an obtainable compound of formula I a radical $R_3$ is converted into a different radical $R_3$, and/or, if desired, an obtainable mixture of isomeric compounds of formula I is separated into the individual isomers, and/or, if desired, an obtainable compound having a salt-forming group is converted into a salt or an obtainable salt is converted into the free compound or into a different salt.

**14.** A compound obtainable by the process according to claim 13.

**Claims for the following Contracting States : ES, GR**

**1.** A process for the preparation of a compound of the formula

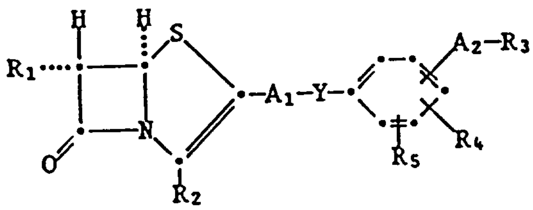

(I)

wherein $R_1$ is hydroxymethyl or 1-hydroxyethyl, $R_2$ is carboxy or esterified carboxy that is cleavable under physiological conditions, $R_3$ is amino, methylamino, dimethylamino, (2-hydroxyethyl)-amino, bis-(2-hydroxyethyl)-amino, (4-pyridyl)-amino, (3-pyridyl)-amino, morpholin-4-yl, piperazin-1-yl, 4-carbamoylpiperazin-1-yl, 3-hydroxy-4-oxo-1,4-dihydropyridin-1-yl, 1H-tetrazol-1-yl, pyridin-4-ylthio or carbamoyl, $R_4$ and $R_5$ are hydrogen, Y is a group -O-, $A_1$ is $C_1$-$C_2$alkylene, and $A_2$ is a direct bond or $C_1$-$C_2$alkylene, a pure optical isomer of a compound of formula I or a mixture of those optical isomers, or a salt of a compound of formula I having a salt-forming group, with the exception of a quaternary ammonium salt, wherein

a) in a compound of the formula

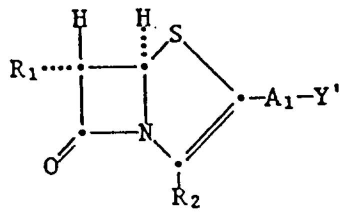

(II),

wherein $R_1$, $R_2$ and $A_1$ are as defined for formula (I) and Y' is a group that can be converted into the radical

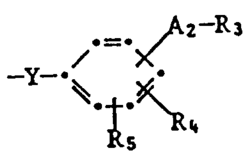

(IIa),

wherein Y, $A_2$, $R_3$, $R_4$ and $R_5$ are as defined for formula (I), the group Y' is converted into the radical

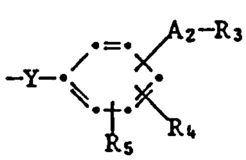

(IIa)

or

b) an ylid compound of the formula

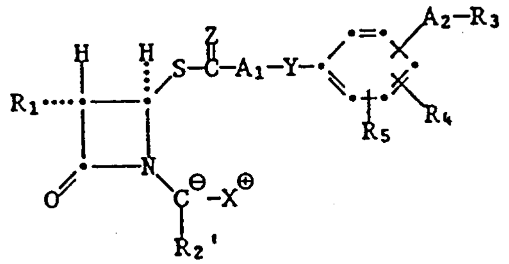

(III),

wherein $R_1$, $R_3$, $R_4$, $R_5$, $A_1$, $A_2$ and Y are as defined for formula I, $R_2'$ is a protected carboxy group, Z is oxygen or sulfur and $X^{\oplus}$ is either a trisubstituted phosphonio group or a diesterified phosphono group together with a cation, is cyclised, or

c) a compound of the formula

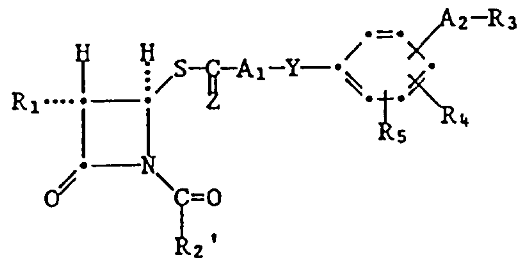

(IV),

wherein $R_1$, $R_3$, $R_4$, $R_5$, $A_1$, $A_2$ and Y are as defined for formula I, Z is oxygen or sulfur and $R_2'$ is a protected carboxy group, is treated with an organic compound of trivalent phosphorus, and, if necessary, in an obtainable compound of formula I a protected functional group is converted into the free functional group, and/or, if desired, in an obtainable compound of formula I a free carboxy group $R_2$ is converted into an esterified carboxy group that is cleavable under physiological conditions, and/or, if desired, in an obtainable compound of formula I a radical $R_3$ is converted into a different radical $R_3$, and/or, if desired, an obtainable mixture of isomeric compounds of formula I is separated into the individual isomers, and/or, if desired, an obtainable compound having a salt-forming group is converted into a salt or an obtainable salt is converted into the free compound or into a different salt.

**2.** A process for the preparation of a compound of formula I according to claim 1, wherein $R_1$ is hydroxymethyl or 1-hydroxyethyl, $R_2$ is carboxy or esterified carboxy that is cleavable under physiological conditions, $R_3$ is amino or carbamoyl, each of $R_4$ and $R_5$ is hydrogen, Y is a group -O-, $A_1$ is $C_1$-$C_2$alkylene, and $A_2$ is a direct bond or $C_1$-$C_2$alkylene, a pure optical isomer of a compound of formula I or a mixture of those optical isomers, or a salt thereof, with the exception of a quaternary ammonium salt.

**3.** A process for the preparation of a compound of formula I according to claim 1, wherein $R_1$ is 1-hydroxyethyl, $R_2$ is carboxy, $R_3$ is amino, methylamino or dimethylamino, each of $R_4$ and $R_5$ is hydrogen, Y is a group -O-, $A_1$ is methylene and $A_2$ is methylene or ethylene, a pure optical isomer of a compound of formula I or a mixture of those optical isomers, or a salt thereof, with the exception of a quaternary ammonium salt.

**4.** A process for the preparation of a pharmaceutically acceptable salt of a compound of formula I according to claim 1.

**5.** A process according to claim 1 for the preparation of (5R,6S)-2-[4-(aminomethyl)-phenoxymethyl]-6-[-(1R)-1-hydroxyethyl]-2-penem-3-carboxylic acid or a pharmaceutically acceptable salt thereof.

6. A process according to claim 1 for the preparation of (5R,6S)-2-[4-aminophenoxymethyl]-6-[(1R)-1-hydroxyethyl]-2-penem-3-carboxylic acid or a pharmaceutically acceptable salt thereof.

7. A process according to claim 1 for the preparation of (5R,6S)-2-[4-(4-morpholinomethyl)-phenoxymethyl]-6-[(1R)-1-hydroxyethyl]-2-penem-3-carboxylic acid or a pharmaceutically acceptable salt thereof.

8. A process according to claim 1 for the preparation of (5R,6S)-2-[4-(dimethylaminomethyl)-phenoxymethyl]-6-[(1R)-1-hydroxyethyl]-2-penem-3-carboxylic acid or a pharmaceutically acceptable salt thereof.

9. A process according to claim 1 for the preparation of (5R,6S)-2-[4-(2-aminoethyl)-phenoxymethyl]-6-[-(1R)-1-hydroxyethyl]-2-penem-3-carboxylic acid or a pharmaceutically acceptable salt thereof.

**Revendications**

**Revendications pour les Etats contractants suivants : AT, BE, CH, DE, FR, GB, IT, LI, LU, NL, SE**

1. Composés de formule

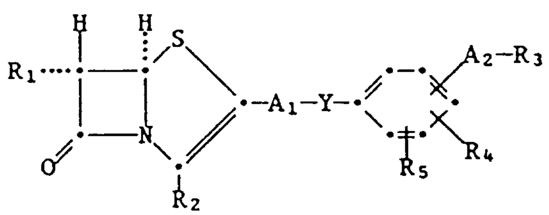

(I),

où $R_1$ est un hydroxyméthyle ou un hydroxyéthyle, $R_2$ représente un carboxy ou un carboxy estérifié clivable dans les conditions physiologiques, $R_3$ est un amino, un méthylamino, un diméthylamino, un (2-hydroxyéthyl)-amino, un bis-(2-hydroxyéthyl)-amino, un (4-pyridyl)-amino, un (3-pyridyl)-amino, un morpholin-4-yle, un pipérazin-1-yle, un 4-carbamoyl-pipérazin-1-yle, un 3-hydroxy-4-oxo-1,4-dihydro-pyridin-1-yle, un 1H-tétrazol-1-yle, un pyridin-4-ylthio ou un carbamoyle, $R_4$ et $R_5$ représentent l'hydrogène, Y représente un groupe -O-, $A_1$ représente un alkylène en $C_1$-$C_2$ et $A_2$ représente une liaison directe ou un alkylène en $C_1$-$C_2$, les isomères optiques purs des composés de formule I et les mélanges de ces isomères optiques et les sels des composés de formule I présentant un groupe salifiable, à l'exception des sels d'ammonium quaternaires.

2. Composés de formule I selon la revendication 1 où $R_1$ est l'hydroxyméthyle ou le 1-hydroxyéthyle, $R_2$ représente un carboxy ou un carboxy estérifié clivable dans les conditions physiologiques, $R_3$ représente un amino ou un carbamoyle, $R_4$ et $R_5$ représentent chacun l'hydrogène, Y représente un groupe -O-, $A_1$ est un alkylène en $C_1$-$C_2$ et $A_2$ représente une liaison directe ou un alkylène en $C_1$-$C_2$, les isomères optiques purs des composés de formule I et les mélanges de ces isomères optiques et leurs sels, à l'exception des sels d'ammonium quaternaires.

3. Composés de formule I selon la revendication 1 où $R_1$ est le 1-hydroxyéthyle, $R_2$ est un carboxy, $R_3$ représente un amino, un méthylamino ou un diméthylamino, $R_4$ et $R_5$ représentent chacun l'hydrogène, Y représente un groupe -O-, $A_1$ est le méthylène et $A_2$ est le méthylène ou l'éthylène, les isomères optiques purs des composés de formule I et les mélanges de ces isomères optiques, et leurs sels, à l'exception des sels d'ammonium quaternaires.

4. Les sels pharmaceutiquement acceptables des composés de formule I selon la revendication 1.

5. L'acide (5R,6S)-2-[4-(aminométhyl)-phénoxyméthyl]-6-[(1R)-1-hydroxyéthyl]-2-pénem-3-carboxylique et ses sels pharmaceutiquement acceptables selon la revendication 1.

6. L'acide (5R,6S)-2-[4-aminophénoxyméthyl]-6-[(1R)-1-hydroxyéthyl]-2-pénem-3-carboxylique et ses sels pharmaceutiquement acceptables selon la revendication 1.

7. L'acide (5R,6S)-2-[4-(4-morpholinométhyl)-phénoxyméthyl]-6-[(1R)-1-hydroxyéthyl]-2-pénem-3-carboxylique et ses sels pharmaceutiquement acceptables selon la revendication 1.

8. L'acide (5R,6S)-2-[4-(diméthylaminométhyl)phénoxyméthyl]-6-[(1R)-1-hydroxyéthyl]-2-pénem-3-carboxylique et ses sels pharmaceutiquement acceptables selon la revendication 1.

9. L'acide (5R,6S)-2-[4-(2-aminoéthyl)-phénoxyméthyl]-6-[(1R)-1-hydroxyéthyl]-2-pénem-3-carboxylique et ses sels pharmaceutiquement acceptables selon la revendication 1.

10. Composé selon la revendication 1 destiné à être utilisé dans un procédé de traitement thérapeutique du corps humain et animal.

11. Préparation pharmaceutique contenant un composé de formule I ou l'un de ses sels pharmaceutiquement acceptables selon la revendication 1, associé à des supports pharmaceutiquement acceptables.

12. Utilisation des composés de formule I ou de leurs sels pharmaceutiquement acceptables selon la revendication 1 pour la préparation des compositions pharmaceutiques destinées à être utilisées comme antibiotiques antibactériens pour le traitement des infections.

13. Procédé pour la préparation des composés de formule I et de leurs sels selon la revendication 1, caractérisé

a) en ce que l'on transforme dans un composé de formule

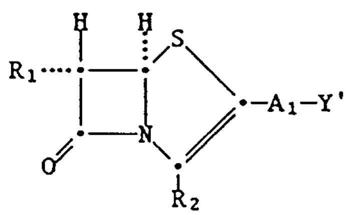

(II),

où $R_1$, $R_2$ et $A_1$ ont les significations indiquées sous la formule (I) et Y' est un groupe transformable en un reste

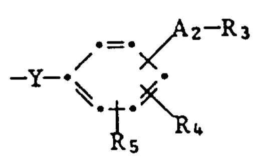

(IIa),

où Y, $A_2$, $R_3$, $R_4$ et $R_5$ ont les significations indiquées sous la formule (I), le groupe Y' en un reste

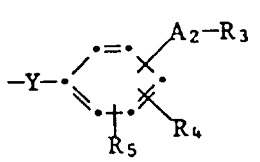

(IIa)

ou

b) en ce que l'on cyclise un composé ylide de formule

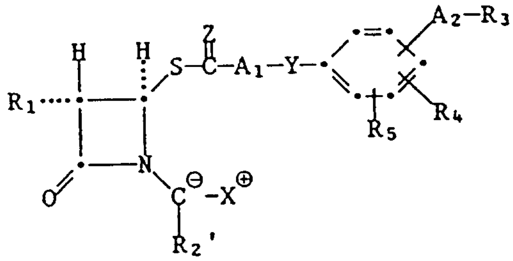

(III),

où $R_1$, $R_3$, $R_4$, $R_5$, $A_1$, $A_2$ et Y ont les significations indiquées sous la formule I, $R_2'$ est un groupe carboxyle protégé, Z représente l'oxygène ou le soufre et $X^{\oplus}$ représente soit un groupe phosphonio trisubstitué, soit un groupe phosphono diestérifié associé à un cation ou

c) en ce que l'on traite un composé de formule

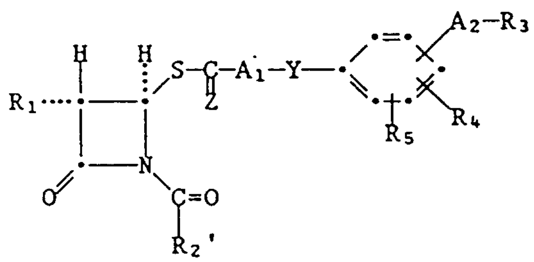

(IV),

où $R_1$, $R_3$, $R_4$, $R_5$, $A_1$, $A_2$ et Y ont les significations indiquées sous la formule I, Z est l'oxygène ou le soufre et $R_2'$ est un groupe carboxyle protégé, avec un composé organique du phosphore trivalent et, si nécessaire, en ce que l'on transforme dans le composé de formule I obtenu un groupe fonctionnel protégé en un groupe fonctionnel libre et/ou, si désiré, en ce que l'on transforme dans le composé de formule I obtenu un groupe carboxyle $R_2$ libre en un groupe carboxyle estérifié clivable dans les conditions physiologiques et/ou, si désiré, en ce que l'on transforme dans le composé de formule (I) obtenu un reste $R_3$ en un autre reste $R_3$ et/ou, si désiré, en ce que l'on sépare le mélange de composés isomères de formule I obtenu en isomères et/ou, si désiré, en ce que l'on transforme le composé obtenu ayant un groupe salifiable en un sel ou le sel obtenu en un composé libre ou en un autre sel.

**14.** Les composés obtenus par le procédé selon la revendication 13.

**Revendications pour les Etats contractants suivants : ES, GR**

**1.** Procédé pour la préparation des composés de formule

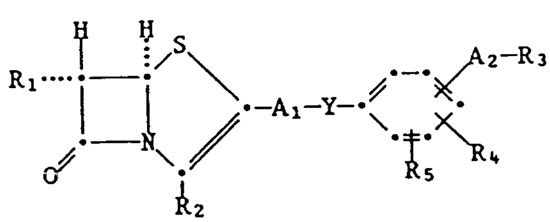

(I),

où $R_1$ est un hydroxyméthyle ou un hydroxyéthyle, $R_2$ représente un carboxy ou un carboxy estérifié clivable dans les conditions physiologiques, $R_3$ est un amino, un méthylamino, un diméthylamino, un (2-hydroxyéthyl)-amino, un bis-(2-hydroxyéthyl)-amino, un (4-pyridyl)-amino, un (3-pyridyl)-amino, un

morpholin-4-yle, un pipérazin-1-yle, un 4-carbamoyl-pipérazin-1-yle, un 3-hydroxy-4-oxo-1,4-dihydro-pyridin-1-yle, un 1H-tétrazol-1-yle, un pyridin-4-ylthio ou un carbamoyle, $R_4$ et $R_5$ représentent l'hydrogène, Y représente un groupe -O-, $A_1$ représente un alkylène en $C_1$-$C_2$ et $A_2$ représente une liaison directe ou un alkylène en $C_1$-$C_2$, des isomères optiques purs des composés de formule I et des mélanges de ces isomères optiques et des sels des composés de formule I présentant un groupe salifiable, à l'exception des sels d'ammonium quaternaires, caractérisé

a) en ce que l'on transforme dans un composé de formule

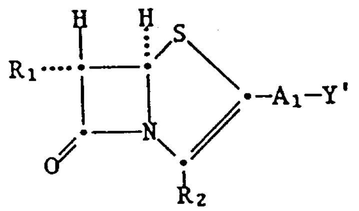

(II),

où $R_1$, $R_2$ et $A_1$ ont les significations indiquées sous la formule (I) et Y' est un groupe transformable en un reste

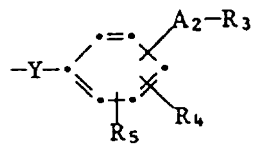

(IIa),

où Y, $A_2$, $R_3$, $R_4$ et $R_5$ ont les significations indiquées sous la formule (I), le groupe Y' en un reste

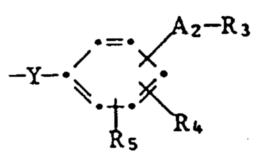

(IIa)

ou

b) en ce que l'on cyclise un composé ylide de formule

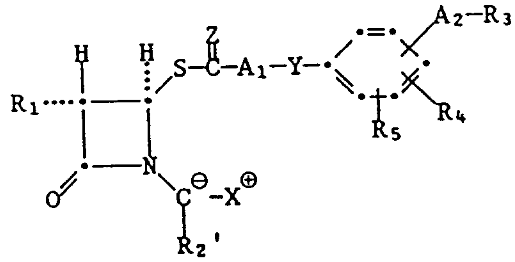

(III),

où $R_1$, $R_3$, $R_4$, $R_5$, $A_1$, $A_2$ et Y ont les significations indiquées sous la formule I, $R_2'$ est un groupe carboxyle protégé, Z représente l'oxygène ou le soufre et $X^{\oplus}$ représente soit un groupe phosphonio trisubstitué, soit un groupe phosphono diestérifié associé à un cation ou

c) en ce que l'on traite un composé de formule

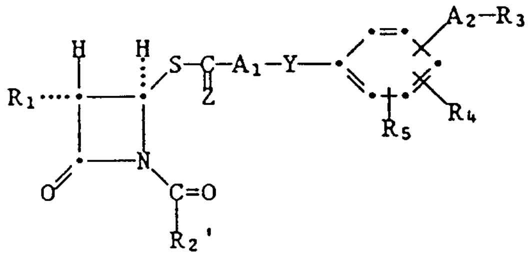

(IV),

où $R_1$, $R_3$, $R_4$, $R_5$, $A_1$, $A_2$ et Y ont les significations indiquées sous la formule I, Z est l'oxygène ou le soufre et $R_2'$ est un groupe carboxyle protégé, avec un composé organique du phosphore trivalent et, si nécessaire, en ce que l'on transforme dans le composé de formule I obtenu un groupe fonctionnel protégé en un groupe fonctionnel libre et/ou, si désiré, en ce que l'on transforme dans le composé de formule I obtenu un groupe carboxyle $R_2$ libre en un groupe carboxyle estérifié clivable dans les conditions physiologiques et/ou, si désiré, en ce que l'on transforme dans le composé de formule I obtenu un reste $R_3$ en un autre reste $R_3$ et/ou, si désiré, en ce que l'on sépare le mélange de composés isomères de formule I obtenu en isomères et/ou, si désiré, en ce que l'on transforme le composé obtenu ayant un groupe salifiable en un sel ou le sel obtenu en un composé libre ou en un autre sel.

**2.** Procédé pour la préparation des composés de formule I selon la revendication 1 où $R_1$ est l'hydroxyméthyle ou le 1-hydroxyéthyle, $R_2$ est un carboxy ou un carboxy estérifié clivable dans les conditions physiologiques, $R_3$ représente un amino ou un carbamoyle, $R_4$ et $R_5$ représentent chacun l'hydrogène, Y représente un groupe -O-, $A_1$ est un alkylène en $C_1$-$C_2$ et $A_2$ représente une liaison directe ou un alkylène en $C_1$-$C_2$ , des isomères optiques purs des composés de formule I et des mélanges de ces isomères optiques et de leurs sels, à l'exception des sels d'ammonium quaternaires.

**3.** Procédé pour la préparation des composés de formule I selon la revendication 1 où $R_1$ est le 1-hydroxyéthyle, $R_2$ est un carboxy, $R_3$ représente un amino, un méthylamino ou un diméthylamino, $R_4$ et $R_5$ représentent chacun l'hydrogène, Y représente un groupe -O-, $A_1$ est le méthylène et $A_2$ est le méthylène ou l'éthylène, des isomères optiques purs des composés de formule I et des mélanges de ces isomères optiques, et de leurs sels, à l'exception des sels d'ammonium quaternaires.

**4.** Procédé pour la préparation des sels pharmaceutiquement acceptables des composés de formule I selon la revendication 1.

**5.** Procédé selon la revendication 1 pour la préparation de l'acide (5R,6S)-2-[4-(aminométhyl)-phénoxyméthyl]-6-[(1R)-1-hydroxyéthyl]-2-pénem-3-carboxylique et de ses sels pharmaceutiquement acceptables.

**6.** Procédé selon la revendication 1 pour la préparation de l'acide (5R,6S)-2-[4-aminophénoxyméthyl]-6-[-(1R)-1-hydroxyéthyl]-2-pénem-3-carboxylique et de ses sels pharmaceutiquement acceptables.

**7.** Procédé selon la revendication 1 pour la préparation de l'acide (5R,6S)-2-[4-(4-morpholinométhyl)-phénoxyméthyl]-6-[(1R)-1-hydroxyéthyl]-2-pénem-3-carboxylique et de ses sels pharmaceutiquement acceptables.

**8.** Procédé selon la revendication 1 pour la préparation de l'acide (5R,6S)-2-[4-(diméthylaminométhyl)-phénoxyméthyl]-6-[(1R)-1-hydroxyéthyl]-2-pénem-3-carboxylique et de ses sels pharmaceutiquement acceptables.

**9.** Procédé selon la revendication 1 pour la préparation de l'acide (5R,6S)-2-[4-(2-aminoéthyl)-phénoxyméthyl]-6-[(1R)-1-hydroxyéthyl]-2-pénem-3-carboxylique et de ses sels pharmaceutiquement acceptables.